# EUROPEAN PATENT SPECIFICATION

(11) **EP 1 686 983 B1**
(45) Date of publication and mention of the grant of the patent: **11.07.2007**
(21) Application number: 04797525.5
(22) Date of filing: 02.11.2004
(51) Int. Cl.: A61K 31/35, A61P 3/00

(54) **USE OF PENTADIENOIC ACID DERIVATES FOR TREATEMENT OF HYPERURICEMIA**
VERWENDUNG VON PENTADIENSÄURE-DERIVATEN ZUR BEHANDLUNG VON HYPERURIKÄMIE
UTILISATION DE DERIVES D'ACIDE PENTADIENOIQUE POUR LE TRAITEMENT DE L'HYPERURICEMIE

(30) Priority: 28.11.2003 EP 03292973; 09.12.2003 US 527773 P
(43) Date of publication of application: 09.08.2006
(73) Proprietor: Merck Patent GmbH, 64293 Darmstadt (DE)
(72) Inventor: BOIZEL, Robert, 38000 Grenoble (FR); FOUQUERAY, Pascale, 75018 Paris (FR); GUERRIER, Daniel, 42600 Lesignieux (FR); ZEILLER, Jean-Jaques, 69008 Lyon (FR); BRUTZKUS, Bertrand, 69005 Lyon (FR)
(86) International application number: PCT/EP2004/012381
(87) International publication number: WO 2005/053676

(56) References cited:
- US-B1- 6 596 758

## Description

### Field of the Invention

The present invention relates to methods for decreasing plasma uric acid levels and treating gout related conditions, using pentadienoic acid derivatives, which have been identified as potent oral hypouricemic agents.

It also relates to the use of these derivatives for preparing medicaments for these methods, and to new medicaments for these purposes.

### Background of the Invention

Uric acid is an end product of purine nucleotide catabolism in humans. Most mammals, but not humans, express the enzyme uricase, which further degrades uric acid to allantoin. Consequently, statistically normal uric acid levels in men and premenopausal women (7 mg per decilitre or 420 *µ*mol/litre and 6 mg per decilitre or 360 *µ*mol/litre respectively) are close to the limits of urate solubility (approximately 7 mg /decilitre at 37°) in vitro, imposing a delicate physiologic urate balance. Uric acid is a weak organic acid. In serum condition of pH 7.40 and temperature 37°C, about 98% of uric acid is ionised as monosodium urate.

Hyperuricemia in humans is common and becomes more common with increased age, diverse pathological states and the use of some medications. Increased serum urate levels may be due to enhanced uric acid production and/or reduced renal excretion of uric acid. Uric acid overproduction may be related to excessive dietary purine intake, specific disease states (malignancy, psoriasis), increased turn over of ATP or inherited enzyme defects. Renal underexcretion of uric acid may be related to defects in the renal handling of uric acid, reduced glomerular filtration of urate or altered reabsorption - secretion by the proximal tubule.

Hyperuricemia is a metabolic disturbance that may lead to gout, which is a commun medical problem, affecting at least 1 percent of men in Western countries. Increased levels of urate may leads to precipitation of urate crystals and tissue deposition of urate, leading to other manifestations of gout: attacks of acute inflammatory arthritis, tophaceous deposition of urate crystals in and around joints, chronic arthritis, deposition of urate crystals in renal parenchyma, and urolithiasis (all, either alone or in combination). The incidence of gouty arthritis is increased 5 fold in subjects with a serum urate level of 7 to 8.9 mg per decilitre and up to 50 fold in subjects with a serum urate level of at least 9 mg per decilitre (530 µmol per liter). Patients with gout may develop renal insufficiency and end stage renal disease. The renal disease, which has been termed "gouty nephropathy", is characterized by a chronic interstitial nephropathy, which is promoted by medullary deposition of monosodium urate. In the vast majority of patients with gout (80 - 90%), increased serum urate levels are related to a diminished renal excretion of uric acid.

On the other hand, secondary hyperuricemia, drug related (i.e. diuretics, immunosuppressive and cytotoxic agents), or related to diverse medical conditions (i.e. various nephropathies, myeloproliferative disorders, conditions associated with insulin resistance and in transplant recipients) may also worsen kidney function leading to chronic and acute renal failure. Overproduction of urate and acid urine also increase the risk of calcium oxalate urolithiasis.

All clinical data and the management of hyperuricemia and gout are supported by references in Oxford Textbook of Clinical Nephrology, The Kidney (Brenner & Rector's), Renal Pathology with Clinical and Functional Correlations, Rheumatology, Principles of Internal Medicine (Harrison's), The Pharmacological Basis of Therapeutics (Goodman & Gilman's) and Terkeltaub R.A. Gout: Clinical Practice.

There has been recent renewed interest in hyperuricemia and its effect on cardiovasucular system. The relation between uric acid and cardiovascular disease has been examined in at least 20 epidemiological and clinical studies. Hyperuricemia is associated with cardiovascular impairment over the long term. Recent epidemiological studies have shown that an elevated uric acid is a common feature of the metabolic syndrome, which confers an increased risk for the development of hypertension, ischemic heart disease and stroke. Whether hyperuricemia is a risk factor for cardiovascular disease (causal role), or only a marker part of the metabolic syndrome, is still debated (Watanabe S *et al*).

The management of gout involves not only treating acute arthritic inflammation and urolithiasis but also lowering urate levels with the goal of preventing recurrent disease and progression. A11 available systemic therapies for acute gouty arthritis (nonsteroidal anti-inflammatory drugs, systemic corticosteroids and colchine) have significant and potentially severe adverse effects, which may contraindicate their use and justify the need of alternative treatments and preventing occurrence or recurrences by lowering plasma urate level, especially in subjects with serum urate level over 9 mg/dL (530 *µ*mol/L). Reduction of serum uric acid below the saturation level may involve any of several therapeutic strategies. The use of xanthine oxidase inhibitors (e.g. allopurinol) results in decreased production of uric acid, but are also associated with side effects sufficiently severe to often warrant discontinuation of therapy, including e.g. induction of hypersensitivity and adverse drug-drug interactions. The use of uricosuric agents increase the excretion of uric acid thereby reducing the plasma concentration. Among these, probenecid, sulfinpyrazone and benzbromarone are the best known. All are not universally available and have many side effects or contraindications. Activators of peroxisome proliferation - activated receptor as uricosuric agents were proposed in WO 00/47209. Certain insulin sensitivity enhancers of this class, such as troglitazone were proposed to prevent or treat hyperuricemia and related disorders in EP-A-0919232. Little is known about possible relationship between hyperuricemia and cardiovascular diseases and the association of hyperuricemia and such diseases was said to be linked to insulin resistance (Wortmann RL, Gout and hyperuricemia Curr. Opin. Rheumatol 2002 May; 14 (3) :281-6) .

Thus, there is a need for further investigation into more potent and safe hypouricemic agents (including uricosuric) to provide new therapeutic treatments offering advantages to existing methods.

Now it was unexpectedly discovered that certain 2,4-pentadienoic acids derivatives, which were disclosed as able to be used in the treatment of dyslipidaemias, artherosclerosis and diabetes, are potent anti-hyperuricemic agents.

These pentadienoic acid derivatives are disclosed in European patent application EP-A-1,140,893 and US patent, 6,596,758 claiming French priority 98 16574 of December 29, 1998.

The present invention provides a method for the prevention and/or the treatment of hyperuricemia and/or associated disorders or diseases by administering to a subject in need thereof, an effective amount of at least one pentadienoic acid derivative of formula (I).

The diseases associated with hyperuricemia to be treated according to the invention comprise one or several of the following : gout, acute inflammatory arthritis, tophaceous deposition of urate crystals in and around joints, chronic arthritis, deposition of urate crystals in renal parenchyma, urolithiasis, and related renal disease also termed gouty nephropaty.

According to the invention the hyperuricemiae able to be treated do not only comprise primary hyperuricemiae but also secondary hyperuricemiae, such as drug related to hyperuricemiae (e.g. by diuretics, immunosuppressive of cytotoxic agents), or hyperuricemiae related to diverse medical conditions (e.g. nephropaties, myeloproliferative disorders, conditions associated with insulin resistance and transplantations).

The subject to be treated according to the method of the invention may or may not suffer from other diseases or disorders such as for example, dyslipidemias, atherosclerosis or diabetes, or diabetes related disorders.

The invention also provides a composition for decreasing serum uric acid levels in a subject by administering to the subject an amount of at least one 2,4-pentadienoic acid derivative of formula (I) effective to reduce the serum uric acid level.

According to a preferred embodiment of the invention, subjects to be treated have serum uric acid levels, before treatment, equal or above 7 mg/dL (420 *µ*mol/L).

Preferably the conditions to be treated are gout or any condition brought about by high levels of uric acid in the joints or kidneys or a serum level over 9 mg/dL (530 µ mol/L).

Preferably the amount to be administered to a subject for decreasing the serum level is an amount which achieves normal uric acid levels.

It is also possible to obtain, if needed, serum level reduction up to 80% from the normal serum level in men or women.

The treatment of the invention is preferably conducted by administering the 2,4-pentadienoic acid derivative of formula (I) by the oral route, but it can also be conducted by any other route including parenteral route such as, for example, by injection or infusion.

The treatment according to the invention is preferably performed by administering the effective amount of 2,4-pentadienoic acid derivative according to formula (I) once or twice per day.

The duration of the treatment can easily be adapted to the conditions of the patient, preferably with the aim to obtain a long term normal acid uric serum level.

The invention also provides the use of a pentadienoic acid derivative of formula (I) for the preparation of a medicament for the prevention or treatment of hyperuricemia and/or one or several of the above mentioned associated disorders or diseases, and/or for reducing the serum uric acid level of a subject.

Preferably the use, according to the invention, allows to prepare medicaments for subjects having serum uric acid levels, before treatment, equal or above 7 mg/dL (420 *µ*mol/L), and more preferably, where the conditions to be treated are gout or any condition brought about by high levels of uric acid in the joints or kidneys or a serum level over 9 mg/dL (530µ mol/L) .

The use according to the invention is preferably conducted for preparing a medicament suitable for administering the 2,4-pentadienoic acid derivative of formula (I) by the oral route, but it can also be conducted by any other route including parenteral route such as, for example, by injection or infusion.

Preferably the use according to the invention allows to prepare a medicament for administering the effective amount of 2,4-pentadienoic acid or derivative according to formula (I) once or twice per day.

The invention also provides new medical compositions for the treatment of hyperuricemiae and/or the above mentioned associated diseases or disorders which comprise, in a vehicle acceptable for a human, an effective amount of at least one 2,4-pentadienoic acid derivative of formula (I).

Preferably this effective amount is substantially lower than the amount needed for the relevant 2,4-pentadienoic acid derivative used in the treatment of dyslipidaemia, atherosclerosis and diabetes.

This effective amount is preferably 50% lower and more preferably 90% or even 95% lower.

For example, the effective amount in a dose for a one day administration for an adult human is comprised between 0.15 and 4 mg/kg of a human body, more preferably between 0.3 and 1.mg/kg.

The compounds used according to the invention correspond to the formula (I) below: 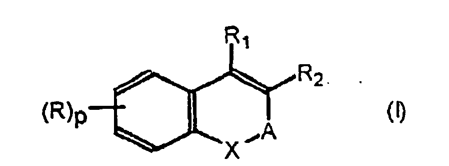 in which:
X represents O or S;
A represents either the divalent radical - (CH₂)ₛ-CO- (CH₂)ₜ- or the divalent radical - (CH₂)ₛ-CR₃R₄-(CH₂) t-
in which radicals s = t = 0 or else one of s and t has the value 0 and the other has the value 1;
R₄ represents a hydrogen atom or a (C₁-C₁₅)alkyl group;
R₁ and R₂ independently represent the Z chain defined below; a hydrogen atom; a (C₁-C₁₈) alkyl group; a (C₂-C₁₈)alkenyl group; a (C₂-C₁₈) alkynyl group; a (C₆-C₁₀) aryl group optionally substituted by a halogen atom, by an optionally halogenated (C₁-C₅)alkyl group or by an optionally halogenated (C₁-C₅)alkoxy group; or a mono- or bicyclic (C₄-C₁₂)heteroaryl group comprising one or more heteroatoms chosen from O, N and S which is optionally substituted by a halogen atom, by an optionally halogenated (C₁-C₅) alkyl group or by an optionally halogenated (C₁-C₅) alkoxy group;
R₃ and R₄ independantly takes any one of the meanings given above for R₁ and R₂, with the exception of the Z chain; or else
R₃ and R₄ together form a (C₂-C₆) alkylene chain optionally substituted by a halogen atom or by optionally halogenated (C₁-C₅)alkoxy;
R is chosen from a halogen atom; a cyano group; a nitro group; a carboxy group; an optionally halogenated (C₁-C₁₈)alkoxycarbonyl group; an Rₐ-CO-NH- or RₐR_{b}N-CO- group [in which Rₐ and R_{b} independently represent optionally halogenated (C₁-C₁₈) alkyl; a hydrogen atom; (C₆-C₁₀)aryl or (C₆-C₁₀) aryl (C₁-C₅) alkyl (where the aryl parts are optionally substituted by a halogen atom, by an optionally halogenated (C₁-C₅) alkyl group or by an optionally halogenated (C₁-C₅)alkoxy group); (C₃-C₁₂) cycloalkyl optionally substituted by a halogen atom, by an optionally halogenated (C₁-C₅)alkyl group or by an optionally halogenated (C₁-C₅)alkoxy group]; an optionally halogenated (C₁-C₁₈)alkyl group; optionally halogenated (C₁-C₁₈)alkoxy; and (C₆-C₁₀) aryl, (C₆-C₁₀) aryl (C₁-C₅) alkyl, (C₆-C₁₀) aryloxy, (C₃ - C₁₂) cyclo-alkyl, (C₃-C₁₂) cycloalkenyl, (C₃-C₁₂) cycloalkyloxy, (C₃-C₁₂) cycloalkenyloxy or (C₆-C₁₀) aryloxycarbonyl in which the aryl, cycloalkyl and cycloalkenyl parts are optionally substituted by a halogen atom, by optionally halogenated (C₁-C₅)alkyl or by optionally halogenated (C₁-C₅)alkoxy; -OH;
p represents 0, 1, 2, 3 or 4;
Z represents the radical: 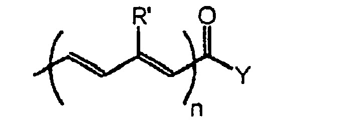 where n is 1 or 2;
The R' groups independently represent a hydrogen atom; a (C₁-C₅)alkyl group; a (C₆-C₁₀) aryl group optionally substituted by a halogen atom, by an optionally halogenated (C₁ - C₅) alkyl group or by optionally halogenated (C₁-C₅) alkoxy; or a mono- or bicyclic (C₄-C₁₂) heteroaryl group comprising one or more heteroatoms chosen from O, N and S which is optionally substituted by a halogen atom, by an optionally halogenated (C₁-C₅) alkyl group or by an optionally halogenated (C₁-C₅)alkoxy group;
Y represents -OH; (C₁-C₅) alkoxy; or the -NR_{c}R_{d} group (in which R_{c} and R_{d} independently represent a hydrogen atom; (C₁-C₅)alkyl; (C₃-C₈) cycloalkyl optionally substituted by a halogen atom, by optionally halogenated (C₁-C₅)alkyl or by optionally halogenated (C₁-C₅) alkoxy; (C₆-C₁₀) aryl optionally substituted by a halogen atom, by optionally halogenated (C₁-C₅)alkyl or by optionally halogenated (C₁-C₅)alkoxy;
Or Y represents glucomic acid 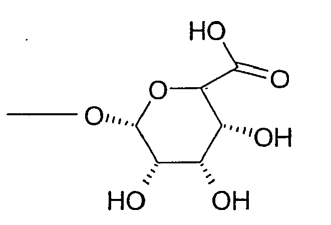 it being understood that one and one alone from R₁ and R₂ represents the Z chain.

The invention is also targeted, depending on the functional groups present in the molecule, at the salts of these compounds with pharmaceutically acceptable acids or bases, and at esters of those compounds.

When the compound of formula (I) comprises an acidic functional group, for example a carboxyl functional group, the latter can form a salt with an inorganic or organic base.

Mention may be made, as example of salts with organic or inorganic bases, of the salts formed with metals and in particular alkali, alkaline earth and transition metals (such as sodium, potassium calcium, magnesium or aluminium) or with bases, such as ammonia or secondary or tertiary amines (such as diethylamine, triethylamine, piperidine, piperazine or morpholine), or with basic amino acids or with osamines (such as meglumine) or with aminoalcohols (such as 3-aminobutanol and 2-aminoethanol).

When the compound of formula (I) comprises a basic functional group, for example a nitrogen atom, the latter can form a salt with an organic or inorganic acid.

The salts with organic or inorganic acids are, for example, the hydrochloride, hydrobromide, sulphate, hydrogensulphate, dihydrogenphosphate, maleate, fumarate, 2-naphthalenesulphonate and para-toluene-sulphonate salts.

The invention also covers the salts which make possible a suitable separation or a suitable crystallization of the compounds of formula (I), such as picric acid, oxalic acid or an optically active acid, for example tartaric acid, dibenzoyltartaric acid, mandelic acid or camphorsulphonic acid.

The formula (I) encompasses all the types of geometric isomers and stereoisomers of the compounds of formula (I).

According to the invention, the term "alkyl" denotes a linear or branched hydrocarbon-comprising radical, such as methyl, ethyl, propyl, isopropyl, butyl, tert-butyl, isobutyl, pentyl, hexyl, heptyl, octyl, nonyl, decyl, undecyl, dodecyl, tridecyl, tetradecyl, pentadecyl, hexadecyl, heptadecyl or octadecyl.

When the alkyl group is substituted by one or more halogen atoms, it is preferable for it to represent perfluoroalkyl and in particular pentafluoroethyl or trifluoromethyl.

The term "alkoxy" denotes an alkyl group as defined above bonded to an oxygen atom. Examples thereof are the methoxy, ethoxy, isopropyloxy, butoxy and hexyloxy radicals.

The term "alkylene group" is understood to mean linear or branched alkylene groups, that is to say bivalent radicals which are linear or branched bivalent alkyl chains.

The term "cycloalkyl" denotes saturated hydrocarbon-comprising groups which can be mono- or polycyclic and comprise from 3 to 12 carbon atoms, preferably from 3 to 8. Preference is more particularly given to monocyclic cycloalkyl groups, such as cyclopropyl, cyclobutyl, cyclopentyl, cyclohexyl, cycloheptyl, cyclooctyl, cyclononyl, cyclodecyl, cycloundecyl and cyclododecyl.

The term "cycloalkenyl" is understood to mean, according to the invention, a cycloalkyl group exhibiting one or more double bonds.

The term "halogen" is understood to mean a fluorine, chlorine, bromine or iodine atom.

The term "aryl" represents a mono- or bicyclic aromatic hydrocarbon-comprising group comprising 6 to 10 carbon atoms, such as phenyl or naphthyl.

The term "mono- or bicyclic heteroaryl" denotes monocyclic or bicyclic aromatic groups comprising one or more endocyclic heteroatoms. Examples thereof are the furyl, thienyl, pyrrolyl, oxazolyl, isoxazolyl, thiazolyl, isothiazolyl, imidazolyl, pyrazolyl, oxadiazolyl, triazolyl, thiadiazolyl, pyridinyl, pyridazinyl, pyrazinyl, triazinyl, indolizinyl, indolyl, isoindolyl, benzofuryl, benzothienyl, indazolyl, benzimidazolyl, benzothiazolyl, purinyl, quinolyl, quinolizinyl, iqoquinolyl, cinnolinyl, phthalazinyl, quinazolinyl, quinoxalinyl, pteridinyl and benzoxepinyl groups.

Preferred heteroaryls comprise from 4 to 10 carbon atoms and from 1 to 2 heteroatoms.

The alkenyl and alkynyl groups can comprise more than one unsaturation.

The alkenyl groups comprise unsaturations of ethylenic type and the alkynyl groups comprise unsaturations of acetylenic type.

The (C₆-C₁₀) aryl, (C₃-C₈) cycloalkyl, heteroaryl and cycloalkenyl groups are optionally substituted. The expression "optionally substituted by a halogen atom, by an optionally halogenated (C₁-C₅)alkyl group or by an optionally halogenated (C₁-C₅)alkoxy group" indicates that the said aryl, cycloalkyl, heteroaryl and cycloalkenyl groups are optionally substituted by one or more substituents chosen from:
- halogen atoms;
- alkyl groups optionally substituted by one or more halogen atoms; and
- alkoxy groups optionally substituted by one or more halogen atoms.

In the same way, the alkylene chain, when it is substituted, can comprise one or more identical or different substituents chosen from halogen atoms and optionally halogenated alkoxy groups.

The expression "optionally halogenated" means, in the context of the invention, optionally substituted by one or more halogen atoms.

In the context of the present invention, the term "benzoxepine" has been used to denote the benzo[b]oxepine structure of formula:

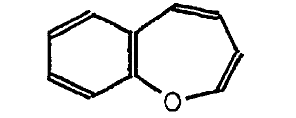

According to the invention, preference is given to the compounds in which A represents the radical:

-(CH₂)ₛ-CR₃R₄- (CH₂) ₜ -

where s, t, R₃ and R₄ are as defined above for the formula (I).

Another preferred group of compounds of formula (I) is composed:
- of the compounds in which:
   X represents O ;
   A represents -CR₃R₄- or -CH₂-CR₃R₄- in which the unsubstituted methylene group is bonded to X;
   R₁ and R₂ independently represent Z; H; (C₁-C₁₅) alkyl; (C₁-C₁₅) alkenyl ; or phenyl optionally substituted by (C₁-C₅) alkyl, (C₁-C₅) alkoxy, a halogen atom or -CF₃;
   R₃ takes any one of the meanings given above for R₁ and R₂, with the exception of Z;
   R is chosen from (C₁-C₉) alkyl; (C₁-C₅) alkoxy; phenyl or phenylcarbonyl optionally substituted by a halogen atom, (C₁-C₅)alkyl, (C₁-C₅)alkoxy, -CF₃ or -OCF₃; a halogen atom; -CF₃ and -OCF₃;
   Z represents the radical: 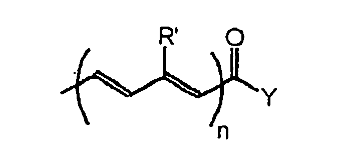 where n represents 1;
      R' represents (C₁-C₅)alkyl.

   Preference is given, among these compounds, to those in which:
   X represents O;
   A represents -CR₃R₄-;
   Z represents 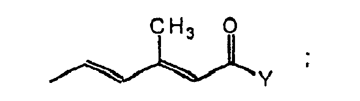
- or alternatively those in which:
   X represents O;
   A represents -CH₂-CR₃R₄- in which the unsubstituted methylene group is bonded to X;
   R₁ and R₂ independently represent Z, a hydrogen atom or (C₁-C₅)alkyl; preferably, R₁ represents Z ; preferably R₂ represents a hydrogen atom.

R₃ and R₄ independently takes any one of the meanings given above for R₁ and R₂, with the exception of Z;

Preferably R₃ and R₄ independently represent a (C₁-C₅) alkyl group, more preferably a methyl, ethyl, isopropyl, propyl and most preferably a methyl.

Z represents

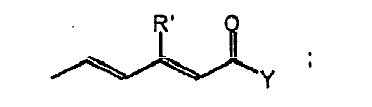

R' represents (C₁-C₅) alkyl, notably a methyl or phenyl, preferably a methyl.

Preferred meanings of Y are:
- OH
- (C₁-C₅)alkoxy; and
- NR_{c}R_{d} where R_{c} and R_{d} are as defined above for the formula (I).

Very preferably, Y represents -OH or -(C₁-C₅) alkoxy, notably a methoxy, ethoxy, isopropryloxy and most preferably ethoxy.

Preferably R represents a (C₁-C₅) alkoxy, notably a methoxy, ethoxy, isopropyloxy, preferably methoxy.

Likewise, it is preferable for p to have the value 0, 1 or 2. Preferably p represents 1 or 2, most preferably 1.

A particularly preferred group of compounds is composed of the compounds in which :
[X represents O ;
A represents -CH₂-CR₃R₄- in which the unsubstituted methylene group is bonded to X ;
R₁ is Z and R₂ is H;
R₃ and R₄ independently represents a (C₁-C₅) alkyl group;
R is a (C₁-C₅) alkoxy;
Z represents 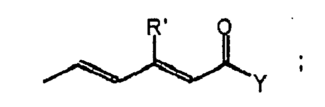 wherein R' represents a methyl or phenyl ; and y represents a (C₁-C₅)alkoxy].

According to a particularly advantageous embodiment of the invention, the compounds of the groups which are preferred defined above are such that p and Y take one of these meanings.

Mention may be made, as example of preferred compounds, of the following compounds:
- (2E, 4E)-5-(2-pentyl-2H-1-benzopyran-3-yl)-3-methyl-penta-2,4-dienoic acid;
- (2Z, 4E)-5-(2-pentyl-2H-1-benzopyran-3-yl)-3-methyl-penta-2,4-dienoic acid;
- (2E, 4E)-5-(2,2-dimethyl-6-methoxy-2H-1-benzopyran-3-yl)-3-methylpenta-2,4-dienoic acid;
- (2E, 4E)-5-(2H-1-benzopyran-3-yl)-3-methylpenta-2,4-dienoic acid;
- (2E, 4E)-5-(2,2-dimethyl-2H-1-benzopyran-3-yl)-3-methylpenta-2,4-dienoic acid;
- (2Z, 4E)-5-(2,2-dimethyl-2H-1-benzopyran-3-yl)-3-methylpenta-2,4-dienoic acid;
- (2E, 4E)-5-[2-(non-6-enyl)-2H-1-benzopyran-3-yl]-3-methylpenta-2,4-dienoic acid;
- (2E, 4E)-5-(4-phenyl-2H-1-benzopyran-3-yl)-3-methylpenta-2,4-dienoic acid;
- (2E, 4E)-5-(6-nonyl-2H-1-benzopyran-3-yl)-3-methyl-penta-2,4-dienoic acid;
- (2E, 4E)-5-(6-phenyl-2H-1-benzopyran-3-yl)-3-methylpenta-2,4-dienoic acid;
- (2E, 4E)-5-(2-nonyl-2H-1-benzopyran-3-yl)-3-methyl-penta-2,4-dienoic acid;
- (2E, 4E)-5-(4-methyl-2H-1-benzopyran-3-yl)-3-methylpenta-2,4-dienoic acid;
- (2Z, 4E)-5-(2H-1-benzopyran-3-yl)-3-methylpenta-2,4-dienoic acid;
- (2E, 4E)-5-(2-undecanyl-2H-1-benzopyran-3-yl)-3-methylpenta-2,4-dienoic acid;
- (2E, 4E)-5-(2-phenyl-2H-1-benzopyran-3-yl)-3-methylpenta-2,4-dienoic acid;
- (2E, 4E)-5-(5-methyl-2,3-dihydrobenzoxepin-4-yl)-3-methylpenta-2,4-dienoic acid;
- (2E, 4E)-5-(3,3-dimethyl-7-methoxy-2,3-dihydrobenzoxepin-5-yl)-3-methylpenta-2,4-dienoic acid; and [sic]
- (2E, 4E)-5-(2,3-dihydrobenzoxepin-4-yl)-3-methylpenta-2,4-dienoic acid;
- (2E, 4E)-5-(3,3-dimethyl-7-methoxy-2,3-dihydrobenzoxepin-5-yl)-3-phenylpenta-2,4-dienoic acid;
- (2Z, 4E)-5-(3,3-dimethyl-7-methoxy-2,3-dihydrobenzoxepin-5-yl)-3-phenylpenta-2,4-dienoic acid;
- (2Z, 4E)-5-(3,3-dimethyl-7-methoxy-2,3-dihydrobenzoxepin-5-yl)-3-methylpenta-2,4-dienoic acid;
- (2E, 4E)-5-(3,3-dimethyl-7,8-dimethoxy-2,3-dihydrobenzo-xepin-5-yl)-3-methylpenta-2,4-dienoic acid;
- (2E, 4E)-5-(3,3-dimethyl-2,3-dihydrobenzoxepin-5-yl)-3-methylpenta-2,4-dienoic acid;
- (2E, 4E)-5-(3,3-dimethyl-2,3-dihydro-7-(para-chlorobenzoyl)benzoxepin-5-yl)-3-methylpenta-2,4-dienoic acid;
- (2E, 4E)-5-(3,3-dimethyl-7-chloro-2,3-dihydrobenzoxepin-5-yl)-3-methylpenta-2,4-dienoic acid;
- (2E, 4E)-5-(3,3-dimethyl-7,8-dichloro-2,3-dihydrobenzoxepin-5-yl)-3-methylpenta-2,4-dienoic acid;
- (2E, 4E)-5-(3,3-dimethyl-7-bromo-2,3-dihydrobenzoxepin-5-yl)-3-methylpenta-2,4-dienoic acid;
- (2E, 4E)-5-(3,3-dimethyl-7-fluoro-8-chloro-2,3-dihydrobenzoxepin-5-yl)-3-methylpenta-2,4-dienoic acid;
- (2E, 4E)-5-(3,3-dimethyl-7-fluoro-2,3-dihydrobenzoxepin-5-yl)-3-methylpenta-2,4-dienoic acid;
- (2E, 4E)-5-(3,3-dimethyl-7-trifluoromethyl-2,3-dihydrobenzoxepin-5-yl)-3-methylpenta-2,4-dienoic acid;
- (2E, 4E)-5-(3,3-dimethyl-7-phenyl-2,3-dihydrobenzoxepin-5-yl)-3-methylpenta-2,4-dienoic acid;
- (2E, 4E)-5-(3,3,7-trimethyl-2,3-dihydrobenzoxepin-5-yl)-3-methylpenta-2,4-dienoic acid;
- (2E, 4E)-5-(3,3-dimethyl-2,3-dihydrobenzoxepin-5-yl)-3-methylpenta-2,4-dienoic acid;
- (2E, 4E)-5-(9-methoxy-3,3-dimethyl-2,3-dihydrobenzoxepin-5-yl)-3-methylpenta-2,4-dienoic acid;
and their pharmaceutically acceptable esters, such as their ethyl esters.

The most preferred compound to be administered in the methods according to the invention, and to be used for the preparation of the medicaments according to the invention, and to be contained as the active principle in the new medicaments is the
(2E,4E)-5-(3,3-dimethyl-7-methoxy-2,3-dihydrobenzoxepin-5-yl)-3-methylpenta-2,4-dienoic acid (compound A), or its pharmaceutically acceptable salts or esters, among which its ethyl ester.

The compounds of formula (I) can be prepared by using one of the processes described in EP-A-1,140,893 or US-B-6,596,758.

The invention additionally relates to pharmaceutical compositions comprising a pharmaceutically effective amount according to the invention of a compound of formula (I) as defined above in combination with one or more pharmaceutically acceptable vehicles.

These compositions can be administered orally in the form of immediate-release or controlled-release granules, hard gelatin capsules or tablets, intravenously in the form of an injectable solution, transdermally in the form of an adhesive transdermal device, or locally in the form of a solution, cream or gel.

A solid composition for oral administration is prepared by addition of a filler and, if appropriate, a binder, a disintegration agent, a lubricant, a colorant or a flavour enhancer to the active principle and by shaping the mixture as a tablet, a coated tablet, a granule, a powder or a capsule.

Examples of fillers encompass lactose, maize starch, sucrose, glucose, sorbitol, crystalline cellulose and silicon dioxide, and examples of binders encompass poly(vinyl alcohol), poly(vinyl ether), ethylcellulose, methycellulose, acacia, gum tragacanth, gelatin, shellac, hydroxypropylcellulose, hydroxypropylmethycellulose, calcium citrate, dextrin and pectin. Examples of lubricants encompass magnesium stearate, talc, polyethylene glycol, silica and hardened vegetable oils. The colorant can be any of those authorized for use in medicaments. Examples of flavour enhancers encompass cocoa powder, mint in herbal form, aromatic powder, mint in oil form, borneol and cinnamon powder. Of course, the tablet or the granule can be suitably coated with sugar, gelatin or the like.

An injectable form comprising the compound of the present invention as active principle is prepared, if appropriate, by mixing the said compound with a pH regulator, a buffer, a suspending agent, a solubilizing agent, a stabilizer, a tonicity agent and/or a preservative and by converting the mixture into a form for intravenous, subcutaneous or intramuscular injection, according to a conventional process. If appropriate, the injectable form obtained can be lyophilized by a conventional process.

Examples of suspending agents encompass methycellulose, polysorbate 80, hydroxyethyl-cellulose, acacia, gum tragacanth powder, sodium carboxymethylcellulose and polyethoxylated sorbitan monolaurate.

Examples of solubilizing agent encompass castor oil solidified with polyoxyethylene, polysorbate 80, nicotinamide, polyethoxylated sorbitan monolaurate and the ethyl ester of castor oil fatty acid.

In addition, the stabilizer encompasses sodium sulphite, sodium metasulphite and ether, while the preservative encompasses methyl p-hydroxybenzoate, ethyl p-hydroxybenzoate, sorbic acid, phenyl, cresol and chlorocresol.

Examples of compounds useful in the present invention are given in Table 1.

**TABLE 1**

| Example | Chemical formula | Characterization physicochemical data |
|---|---|---|
| 3a | 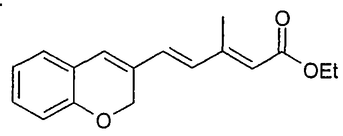 | M.p. = 110-112°C |
| 3b | 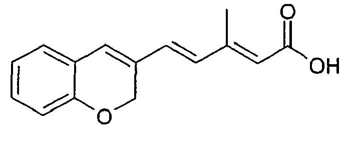 | M.p. = 226-228°C ¹H NMR (d6-DMSO, 300 MHz) δ (ppm): 2.4 (3H, s), 5.2 (2H, s), 6.0 (1H, s), 6.6 (1H, d, J = 16 Hz), 7.1-6.9 (4H, m), 7.3-7.2 (2H, m) |
| 4a | 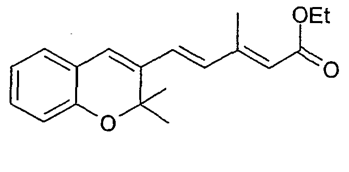 | ¹H NMR (CDCl₃, 300 MHz) δ (ppm): 1.52 (3H, t, J = 7.1 Hz), 1.74 (6H, s), 2.56 (3H, d, J = 1.1 Hz), 4.41 (2H, q, J = 7 .1 Hz), 6.09 (1H, s), from 6.66 to 7.36 (7H, m). |
| 4b | 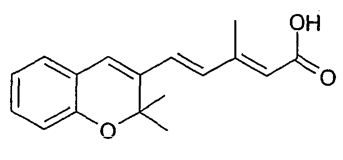 | M.p. = 164-166°C ¹H NMR (CDCl₃, 300 MHz) δ (ppm): 1.38 (6H, s), 2.5 (3H, s), 6 .03 (1H, s), 6.68-7.26 (7H, m). |
| 5a | 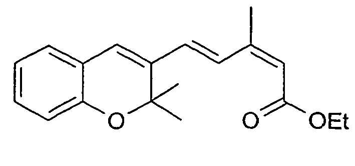 | ¹H NMR (CDCl₃, 300 MHz) δ (ppm): 1.41 (3H, t, J = 7.14 Hz), 1.68 (6H, s), 2.16 (3H, d, J = 1.2 Hz), 4.3 (2H, q, J = 7.13 Hz), 5.82 (1H, s), 6.58 (1H, d, J = 16.35 Hz), from 6.79 to 7.24 (5H, m), 8.3 (1H, d, J = 16.2 Hz). |
| 5b | 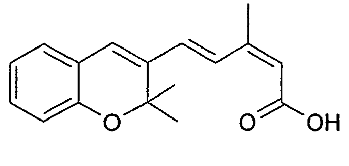 | M.p. = 176°C ¹H NMR (CDCl₃, 300 MHz) δ (ppm): 1.81 (6H, s) 2.35 (3H, s), 6 (1H, s), 6.77 (1H, d, J = 16.2 Hz), 6.93 (1H, s), from 7.02 to 7.4 (4H, m) 8.37 (1H, d, J = 16.2 Hz). |
| 6a | 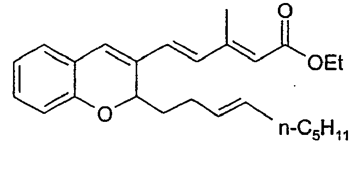 | ¹H NMR (CDCl₃, 300 MHz) δ (ppm): 0.8 (3H, t, J - 7 Hz), 1.2-2.3 (15H, m), 2.3 (3H, s), 4.1 (2H, q, J = 7 Hz), 5.0 (1H, d, J = 14 Hz), (2H, m), 5.8 (1H, s), 6.1 (1H, d, J = 16 Hz), 6.4 (1H, s), 6.5 (1H, d, J = 16 Hz), (4H, m). |
| 6b | 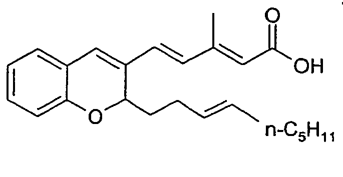 | M.p. = 120-122°C ¹H NMR (CDCl₃, 300 MHz) δ (ppm): 0.8 (3H, t, J = 6.5 Hz), 2.3-0.8 (12H, m), 2.3 (3H, s), 5.1 (1H, d, J = 10 Hz), (2H, m), 5.8 (1H, s), 6.2 (1H, d, J = 16 Hz), 6.5 (1H, s), 6.6 (1H, d, J = 16 Hz), (4H, m). |
| 7a | 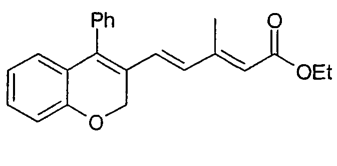 | ¹H NMR (CDCl₃, 300 MHz) δ (ppm): 1.2 (3H, m), 2 (3H, s), from 4.04 (2H, m), 5 (2H, s), 5.7 (1H, s), 6.1 (1H, d, J = 16.3 Hz), from 6.47 to 7.39 (10H, m). |
| 7b | 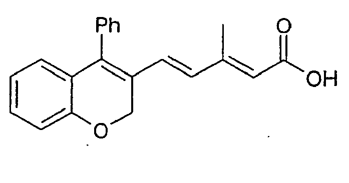 | M.p. = 258-260°C ¹H NMR (d6-DMSO, 300 MHz) δ (ppm): 1.73 (3H, s), 3.125 (1H, TFA exchangeable), 4.89 (2H, s), 5.67 (1H, s) 6.26 to 6.49 (3H, m), from 6.63 to 6.73 (2H, m), from 6.97 to 7. m), from 7.25 to 7.31 (3H, m). |
| 8a | 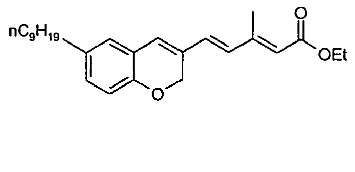 | ¹H NMR (CDCl₃, 300 MHz) δ (ppm): 0.6-1.6 (22H, m), 2.3 (3H, s), 4.1 (2H, q, J = 7 Hz), 4.9 (2H, s), 5.8 (1H, s), 6.1 (1H, d, Hz), 6.5 (1H, s), 6.6 (1H, d, J = 16 Hz), 6.7-7.0 (4H, m). |
| 8b | 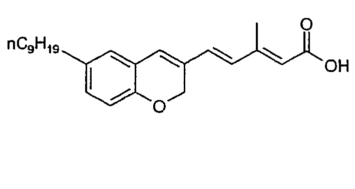 | M.p. = 161-164°C ¹H NMR (CDCl₃, 300 MHz) δ (ppm): 0.5-1.6 (19H, m), 2.3 (3H, s), 4.9 (2H, s), 5.9 (1H, s), 6.1 (1H, d, J = 16 Hz), 6.6 (1H, s), 6.7-6.6 (2H, m), 7.1-6.8 (2H, m) . |
| 9a | 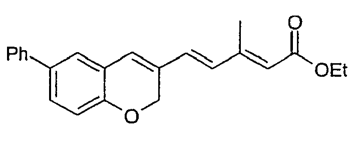 | ¹H NMR (CDCl₃, 300 MHz) δ (ppm): 0.8 (3H, m), 2.3 (3H, s), 4.1 (2H, m), 5.0 (2H, s), 5.8 (1H, s), 6.1 (1H, d, J = 16 Hz), 6.5-6.7 (2H, m), 6.8-6.9 (1H, m), 7.1-7.5 (7H, m) |
| 9b | 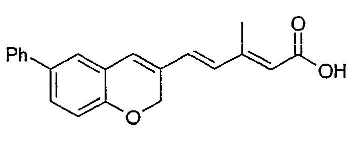 | ¹H NMR (d6-DMSO, 300 MHz) δ (ppm): 2.1 (3H, s), 4.9 (2H, s), 5.8 (1H, s) 6.34 (1H, d, J = 16 Hz), 6.8-6.6 (3H, m), 7.5-7.2 (7H, m). |
| 10a | 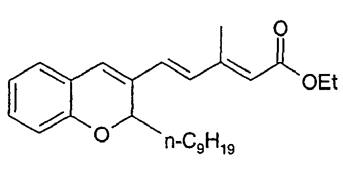 | ¹H NMR (CDCl₃, 300 MHz) δ (ppm) : 0.8 (3H, t, J = 7 Hz), 1.2-1.7 (19H, m), 2.3 (3H, s), 4.1 (2H, q, J = 7 Hz), 5.0 (1H, d, J = 10 Hz), 5.8 (1H, s), 6.1 (1H, d, J = 16 Hz), 6.4 (1H, s), 6.6 (1H, d, J = 16 Hz), 6.8-7.2 (4H, m). |
| 10b | 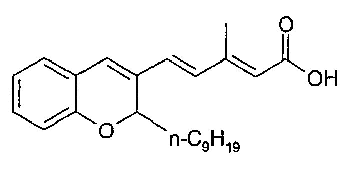 | M.p. = 104-106°C ¹H NMR (CDCl₃, 300 MHz) δ (ppm) : 0.8 (3H, m), (16H, m), 5.0 (1H, d, Hz), 5.8 (1H, s), 6.2 (1H, d, J = 16 Hz), 6.5 (1H, s), 6.6 (1H, d, J = 16 Hz), 6.9-6.8 (2H, m), 7.1-7.0 (2H, m) |
| 11a | 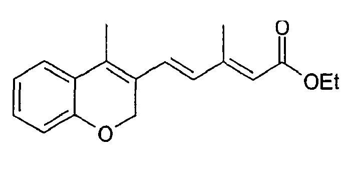 | ¹H NMR (CDCl₃, 300 MHz) δ (ppm): 1.2 (3H, t, J = 7 Hz), 2.0 (3H, s), 2.3 (3H, s), 4.1 (2H, q, J = 7 Hz), 4.8 (2H, s), 5.8 (1H, s), 6.1 (1H, d, J = 16 Hz), (5H, m). |
| 11b | 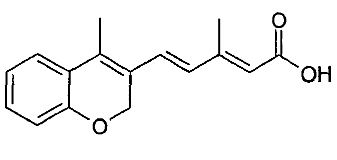 | M.p. = 216-218°C ¹H NMR (CDCl₃, 300 MHz) δ (ppm) : 2.15 (3H, s), 2.3 (3H, s), 4.8 (2H, s), 5.8 (1H, s), 6.2 (1H, d, J = 16 Hz), 6.9-6.8 (2H, m), 7.3-7.0 (3H, m). |
| 12a | 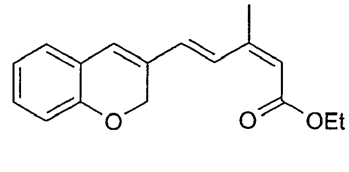 | ¹H NMR (CDCl₃, 300 MHz) δ (ppm): 1.2 (3H, t, J = 7 Hz), 2.0 (3H, s), 4. 1 (2H, q, J = 7 Hz), 5.0 (2H, s), 5.7 (1H, s) (1H, s), 6.5 (1H, s), 6.6 (1H, d, Hz), 6.7-7.2 (4H, m), 7.7 (1H, d, J = 16 Hz). |
| 12b | 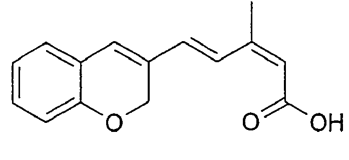 | M.p. = 224-226°C ¹H NMR (d6-DMSO, 300 MHz) δ (ppm): 2.1 (3H, s), 5.0 (2H, s), 5.8 (1H, s), 7.0-6.8 (4H, m), 7.2-7.18 (2H, m), 7.7 (1H, d, J = 16 Hz). |
| 13a | 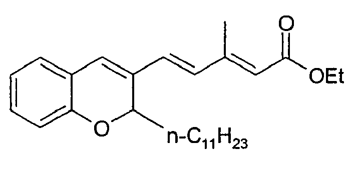 | ¹H NMR (CDCl₃, 300 MHz) δ (ppm): 0.8 (3H, t, J = 7 Hz), 1.2-1.8 (23H, m), 2.3 (3H, s), 4.1 (2H, q, J = 7 Hz), 5.0 (1H, d, J = 10 Hz), 5.8 (1H, s), 6.1 (1H, d, J = 16 Hz), 6.4 (1H, s), 6.6 (1H, d, J = 16 Hz), 6.8-7.1 (4H, m). |
| 13b | 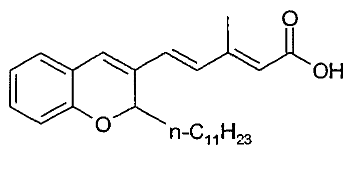 | M.p. = 115-117°C ¹H NMR (CDCl₃, 300 MHz) δ (ppm): 0.8 (3H, t, J = 6.5 Hz), 1.8-1.2 (20H, m), 2.3 (3H, s), 5.0 (1H, d, J = 10 Hz), 5.8 (1H, s), 6.2 (1H, d, J = 16 Hz), 6.5 (1H, s), 6.6 (1H, d, J = 16 Hz), 6.8 (2H, m), 7.0 (1H, d, J = 8 Hz), 7.1 (1H, t, J = 8 Hz). |
| 14a | 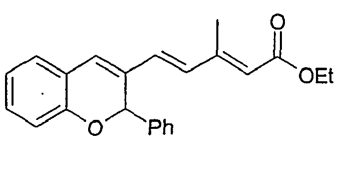 | ¹H NMR (CDCl₃, 300 MHz) δ (ppm): 1.2 (3H, t, J = 7 Hz), 2.2 (3H, s), 4.1 (2H, q, J = 7 Hz), 5.6 (1H, s), 6.0 (1H, d, J = 6 Hz), 6.1 (1H, s) 6.7 (1H, d, J = 6 Hz), 6.8 (1H, s), 6.8-7 (9H, m) |
| 14b | 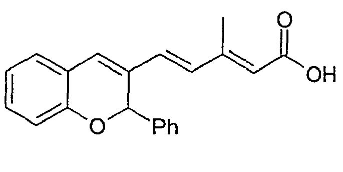 | M.p. = 200-202°C ¹H NMR (d6-DMSO, 300 MHz) δ (ppm) : 2.2 (3H, s), 5.8 (1H, s), 6.36 (1H, s), 6.4 (1H, d, J = 16 Hz), 6.8 (1H, d, J = 8 Hz), 7.4-6.9 (10H, m) |

Other coumpound examples are given in the following Table 2.

**TABLE 2**

| Example | Chemical formula | Characterization physicochemical data |
|---|---|---|
| 18 | 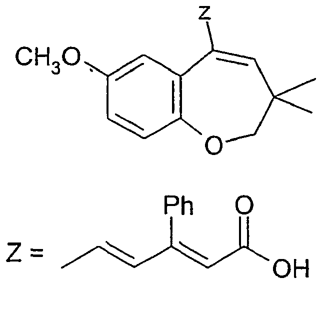 | M.p. 182-184°C ¹H NMR (CDCl₃, 300 MHz) of the corresponding ethyl ester δ (ppm): 7.4-7.1 (5H, m), 6.85-6.8 (1H, d, J = 8.73), 6.7 to 6.45 (3H, m), 6.2 to 6.15 (1H, d, J = 15.35 Hz), 5.95 (1H, s), 5.9 (1H, s), 3.95 (2H, q), 3.75 (2H, s), 3.65 (3H, s), 1.1 (6H, s), 1 (3H, t) |
| 19 | 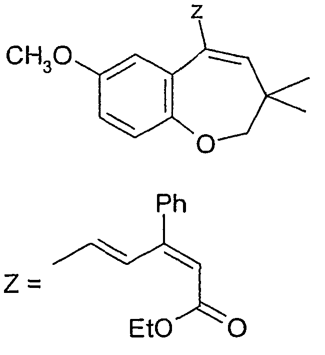 | ¹H NMR (CDCl₃, 300 MHz) δ (ppm): 8 (1H, d, J = 15.69 Hz), 7.3 (5H, s), 6.85-6.8 (1H, d, J = 8.48 Hz), 6.6 (2H, m), 6.4-6.35 (1H, d, J = 15.65 Hz), 6.1 (1H, s), 5.7 (1H, s), 4.15 (2H, q), 3.8 (2H, s), 3.65 (3H, s), 1.25 (3H, t), 1.1 (6H, s) |
| 20a | 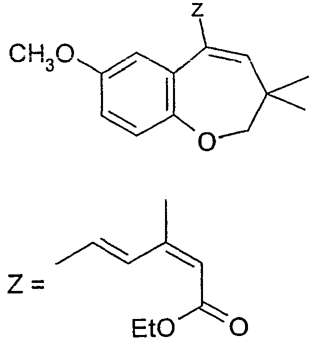 | ¹H NMR (CDCl₃, 300 MHz) δ (ppm) : 7.92 (1H, d, J = 15.79), 6.7-6.95 (4H, m), 6.08 (1H, s), 5.7 (1H, s), 4.16 (2H, q), 3.84 (2H, s), 3.75 (3H, s), 2.07 (3H, s), 1.28 (3H, t), 1.15 (6H, s) |
| 20b | 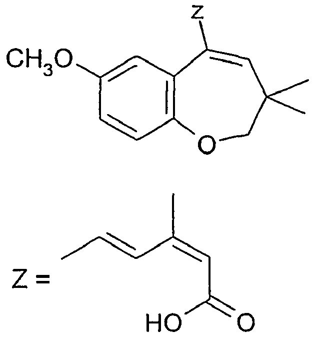 | IR (cm⁻¹) = 2975, 1683, 1493, 1244 ¹H NMR (CDCl₃, 300 MHz) δ (ppm): 7.8-7.9 (1H, d, J = 15.66 Hz), 6.9 (1H, d), 6.8-6.6 (3H, m), 6 (1H, s), 5.65 (1H, s), 3.8 (2H, s), 3.7 (3H, s), 2.05 (3H, s), 1.1 (6H, s). |
| 21a | 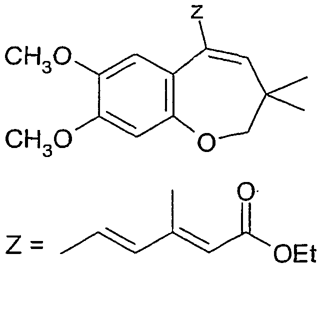 | ¹H NMR (CDCl₃, 300 MHz) δ (ppm): 6.73-6.62 (2H, m), 6.52 (1H, s), 6.40-6.37 (1H, d, J = 15.4 Hz), 5.80 (1H, s), 5.77 (1H, s), 4.15-4.07 (2H, m), 3.82 (2H, s), 3.75 (3H, s), 3.74 (3H, s), 2.3 (3H, s), 1.25-1.19 (3H, m), 1.08 (6H, s) |
| 21b | 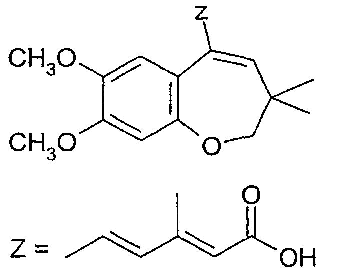 | M.p. = 181-183°C |
| 22a | 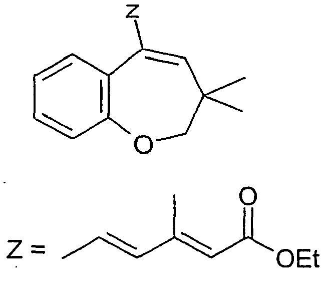 | ¹H NMR (CDCl₃, 300 MHz) δ (ppm): 7.18-6.94 (4H, m), 6.84 (1H, d, J = 15.4 Hz), 6.36 (1H, d, J = 15.4 Hz), 5.90 (1H, s), 5.77 (1H, s), 4.15-4.07 (2H, m), 3 . 83 (2H, s), 2.30 (3H, s), 1.24-1.16 (3H, m), 1.09 (6H, s) |
| 22b | 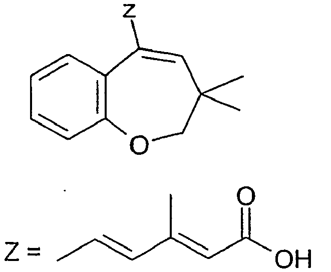 | M.p. = 178-180°C |
| 23a | 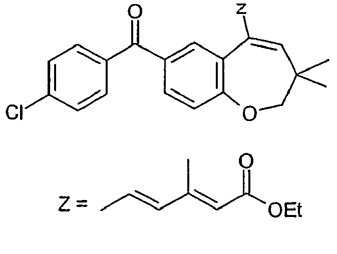 | ¹H NMR (CDCl₃, 300 MHz) δ (ppm): 7.70-7.54 (4H, m), 7.39-7.34 (2H, m), 7.02 (1H, d, J = 7.9 Hz), 6.67 (1H, d, J = 15.4 Hz), 6.36 (1H, d, J = 15.4 Hz), 6.09 (1H, s), 5.76 (1H, s), 4.15-4.03 (2H, m), 3.29 (2H, s), 1.96 (3H, s), 1.24-1.20 (3H, m), 1.16 (6H, s) |
| 23b | 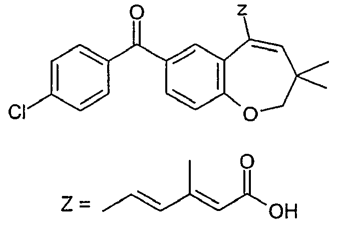 | M.p. = 206-208°C |
| 24a | 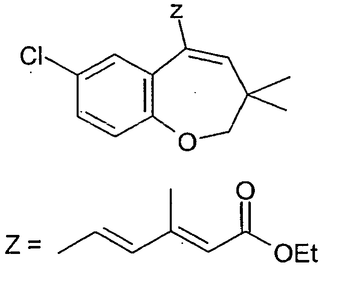 | ¹H NMR (CDCl₃, 300 MHz) δ (ppm): 7.18-7.03 (2H, m), 6.89 (1H, d, J = 8.5 Hz), 6.64 (1H, d, J = 15.4 Hz), 6.35 (1H, d, J = 15.4 Hz), 5.93 (1H, s), 5.78 (1H, s), 4.16-4.08 (2H, m), 3.80 (2H, s), 2.31 (3H, s), 1.25-1.18 (3H, m), 1.08 (6H, s) |
| 24b | 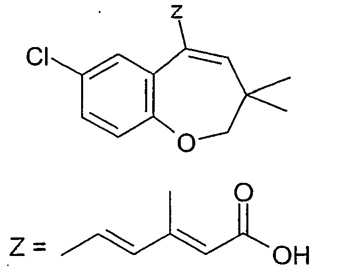 | M.p. = 177-179°C |
| 25 | 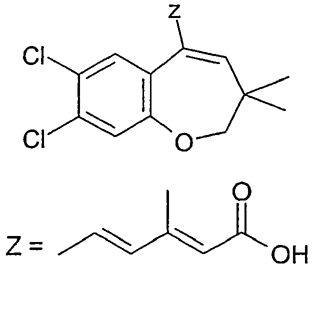 | M.p. = 180°C ¹H NMR (CDCl₃, 300 MHz) of the corresponding ethyl ester δ (ppm): 7.25 (1H, s), 7 (1H, s), 6.6 (1H, d), 6.3 (1H, d), 5.9 (1H, s), 5.8 (1H, s), 4.15 (2H,m), 3.8 (2H, s), 2.3 (3H, s), 1.2 (3H, t), 1.1 (6H, s). |
| 26a | 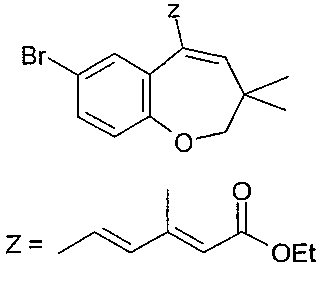 | ¹H NMR (CDCl₃, 300 ·MHz) δ (ppm): 7.29-6.81 (3H, m), 6.7 (1H, d, J = 15.4 Hz), 6.35 (1H, d, J = 15.4 Hz), 5.92 (1H, s), 5.79 (1H, s), 4.10 (2H, m), 3.8 (2H, s), 2.31 (3H, s), 1.21 (3H, m), 1.16 (6H, s) |
| 26b | 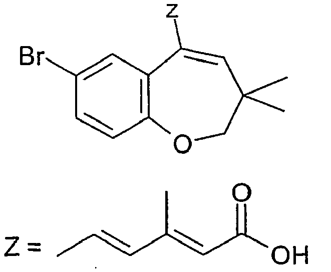 | M.p. = 164-165°C |
| 27 | 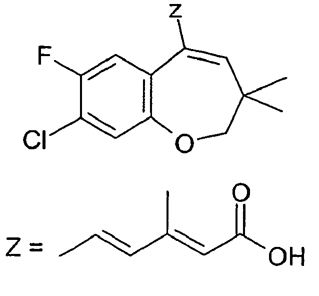 | M.p. = 200°C ¹H NMR (CDCl₃, 300 MHz) δ (ppm) of the corresponding ethyl ester : 7 (2H, m), 6.6 (1H, d, J = d, J = 15.45 Hz), 6.3 (1H, d, J = d, j = 15.42 Hz) 6 (1H, s), s), s), 5.8 (1H, s), 4.1 (2H, m), 3.8 m), 3.8 (2H, s), 2.3 (3H, s), 1.1 s), 1.1 (6H, s) |
| 28a | 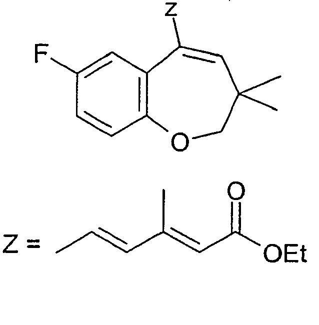 | ¹H NMR (CDCl₃, 300 MHz) δ (ppm): 7.19-6.78 (3H,m),6.64), 6.64 (1H, d, J = 15.4 Hz), 6.34 6.34 (1H, d, J = 15.4 Hz), 5.93 5.93 (1H, s), 5.78 (1H, s), 4.15-4.03 (2H, m), 3.80 (2H, s), 2.30 (3H, s), 1.25-1.20 (3H, m), 1.09 (6H, s) |
| 28b | 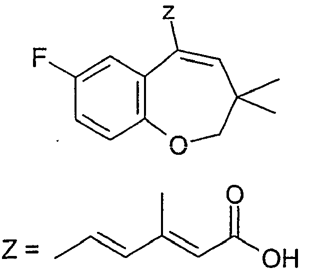 | M.p. = 193-195°C |
| 29a | 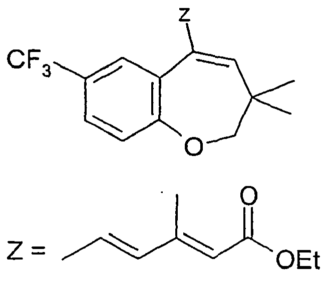 | ¹H NMR (CDCl₃, 300 MHz) δ (ppm): 7.45-7.34 (2H,m), 7.02 (1H, d, J = 8.1 Hz), 6.66 (1H, d, J = 15.4 Hz), 6. 37 (1H, d, J = 15.4 Hz), 5.98 (1H, s), 5 s), 4.16-4.09 (2H, m), 3.85 (2H, s), 2.30 (3H, s), 1.25-1.16 (3H, m), 1.11 (6H, s) |
| 29b | 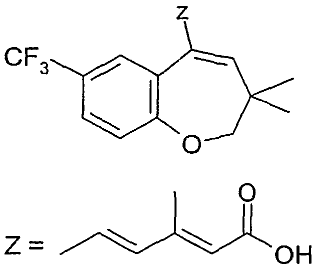 | M.p. = 163-165°C |
| 30a | 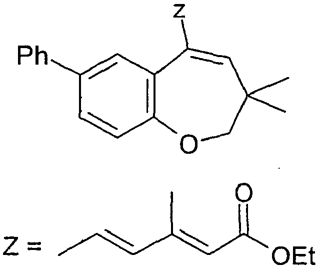 | ¹H NMR (CDCl₃, 300 MHz) δ (ppm): 7-7.6 (8H, m), 6.9 (1H, d, J = 15.47 Hz), 6.5 (1H, d, J = 15.43 Hz), 6 (1H, s), 5.9 (1H, s), 4 (2H, m), 3.8 (2H, s), 2.24 (3H, s), 1.1 (3H, t), 1.01 (6H, s) |
| 30b | 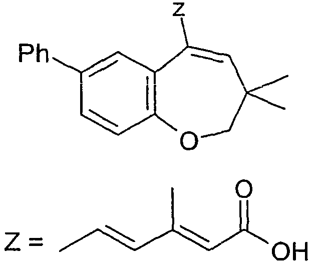 | M.p. = 206-208°C |
| 31 | 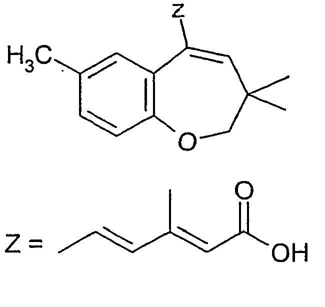 | ¹H NMR (CDCl₃, 300 MHz) δ (ppm): 12.2 (1H, s, exchangeable with CF₃COOD), 7.17-7.06 (2H, m), 6.86-6.97 (2H, m), 6.57 (1H, d, J = 15.4 Hz), 6.10 (1H, s), 5.94 (1H, s), 3 . 89 (2H, s), 2.37 (3H, s), 2.32 (3H, s), 1.17 (6H, s) |
| 32a | 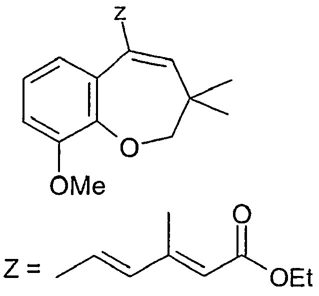 | M.p. = 94°C ¹H NMR (CDCl₃, 300 MHz) δ (ppm): 6.88-6.68 (4H, m), 6.35 (1H, d, J = 15.44 Hz), 5.92 (1H, s), 5.76 (1H, s), 4.10 (2H, m), 3.9 (2H, s), 3.83 (3H, s), 2.3 (3H, s), 1.22 (3H, m), 1.1 (6H, s) |
| 32b | 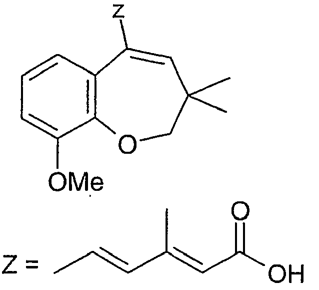 | M.p. = 180-184°C ¹H NMR (CDCl₃, 300 MHz) δ (ppm) : 7.15-6.94 (4H, m), 6.59 (1H, d, J = 15.35 Hz), 6.15 (1H, s), 6.0 (1H, s), 4.11 (2H, s), 4.0 (3H, s), 2.51 (3H, s), 1.3 (6H, s) |
| 33 | 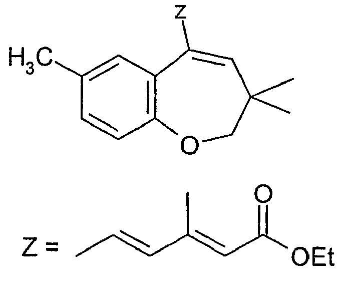 | ¹H NMR (CDCl₃, 300 MHz) δ (ppm): 7. 1-6. 8 (3H, m), 6.72 (1H, d, J = 16 Hz), 6 .35 (1H, d, J = 15.4 Hz), 5.87 (1H, s), 5.77 (1H, s), 4.15-4.08 (2H, m), 3 . 80 (2H, s), 2.30 (3H, s), 2.20 (3H, s), 1.25-1.18 (3H, m), 1.08 (6H, s) |

| Ex | Chemical formula | Nomenclature |
|---|---|---|
| 34A | 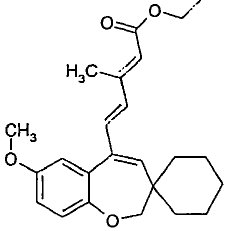 | (2E, 4E)-5-(Spiro[(7-methoxy-2,3-dihydrobenzo[b]-oxepine)-3,1'-cyclohexane]-5-yl)-3-methyl-penta-2,4-dienoic acid ethyl ester |
| 34B | 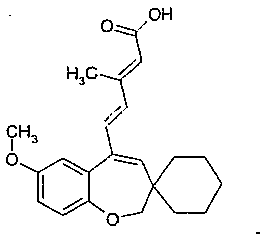 | (2E, 4E)-5-(Spiro[(7-methoxy-2,3-dihydrobenzo[b]-oxepine)-3,1'-cyclohexane]-5-yl)-3-methyl-penta-2,4-dienoic acid. |
| 35A | 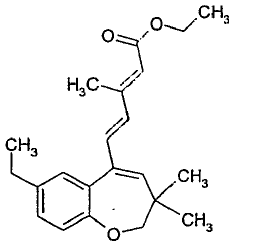 | (2E, 4E)-5-(7-Ethyl-3,3-dimethyl-2,3-dihydrobenzo[b]-oxepine-5-yl)-3-methylpenta-2,4-dienoic acid ethyl ester |
| 35B | 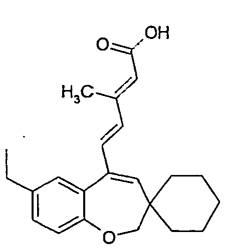 | (2E, 4E)-5-(7-Ethyl-3,3-dimethyl-2,3-dihydrobenzo[b]-oxepin-5-yl)-3-methylpenta-2,4-dienoic acid |
| 36A | 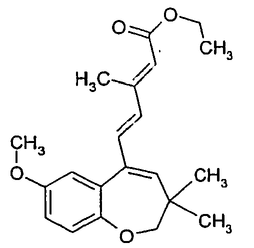 | (2E, 4E)-5-(7-(4-Methoxyphenyl)-3,3-dimethyl-2,3-dihydrobenzo[b]-oxepin-5-yl]-3-methylpenta-2,4-dienoic acid ethyl ester |
| 36B | 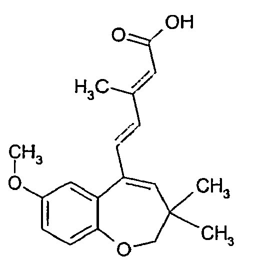 | (2E, 4E) -5- (7- (4-Methoxyphenyl) -3, 3-dimethyl-2,3-dihydrobenzo[b]-oxepin-5-yl]-3-methylpenta-2,4-dienoic acid |
| 37A | 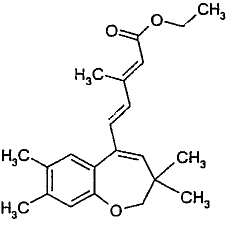 | (2E, 4E)-3-Methyl-5-(3,3,7,8-tetramethyl-2,3-dihydrobenzo[b]-oxepin-5-yl)penta-2,4-dienoic acid ethyl ester |
| 37B | 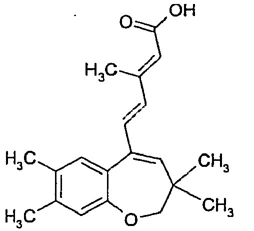 | 3-Methyl-5-(3,3,7,8-tetramethyl-2,3-dihydrobenzo[b]-oxepin-5-yl)penta-2,4-dienoic acid |
| 38A | 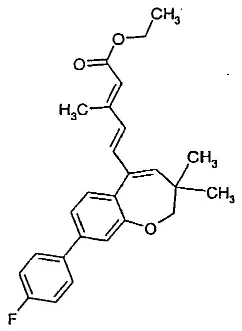 | (2E, 4E)-5-[8-(4-Fluorophenyl)-3,3-dimethyl-2, 3-dihydrobenzo[b]-oxepin-5-yl]-3-methylpenta-2,4-dienoic acid ethyl ester |
| 38B | 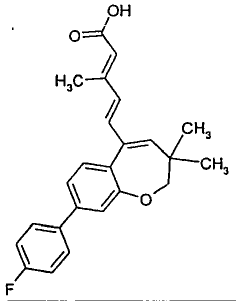 | (2E, 4E)-5-[8-(4-Fluorophenyl)-3,3-dimethyl-2,3-dihydrobenzo[b]-oxepin-5-yl]-3-methylpenta-2,4-dienoic acid |
| 39A | 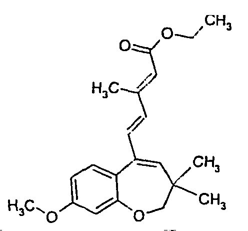 | (2E, 4E)-5-(8-Methoxy-3,3-dimethyl-2,3-dihydrobenzo[b]-oxepin-5yl)-3-methylpenta-2,4-dienoic acid ethyl ester |
| 39B | 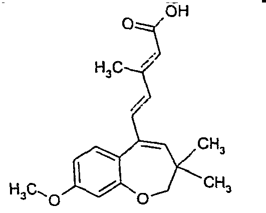 | (2E, 4E)-5-(8-Methoxy-3,3-dimethyl-2,3-dihydrobenzo[b]-oxepin-5-yl)-3-methylpenta-2,4-dienoic acid |
| 40A | 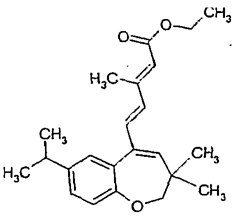 | (2E, 4E)-5-(7-Isopropyl-3,3-dimethyl-2, 3-dihydrobenzo[b]-oxepin-5-yl)-3-methylpenta-2,4-dienoic acid ethyl ester. |
| 40B | 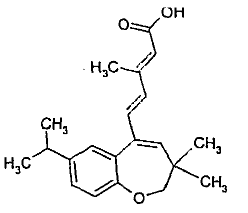 | (2E, 4E)-5-(7-Isopropyl-3,3-dimethyl-2,3-dihydrobenzo[b]-oxepin-5-yl)-3-methylpenta-2,4-dienoic acid. |
| 41 | 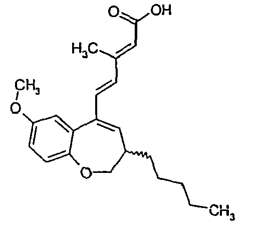 | (2E, 4E)-5-(7-Methoxy-3-pentyl-2,3-dihydrobenzo [b] -oxepin-5-yl)-3-methylpenta-2,4-dienoic acid. |
| 42A | 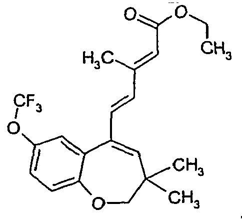 | (2E, 4E)-5-(3,3-Dimethyl-7-trifluoromethoxy-2,3-dihydrobenzo[b]-oxepin-5-yl)-3-methylpenta-2,4-dienoic acid ethyl ester |
| 42B | 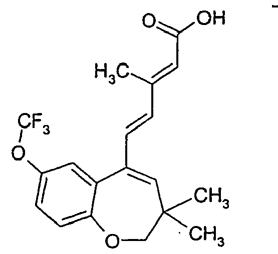 | (2E, 4E)-5-(3,3-Dimethyl-7-trifluoromethoxy-2,3-dihydrobenzo [b]-oxepin-5-yl)-3-methylpenta-2,4-dienoic acid |
| 43A | 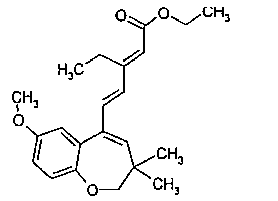 | (2E, 4E)-3-Ethyl-5-(7-methoxy-3,3-dimethyl-2,3-dihydrobenzo[b]-oxepin-5-yl)penta-2,4-dienoic acid ethyl ester |
| 43B | 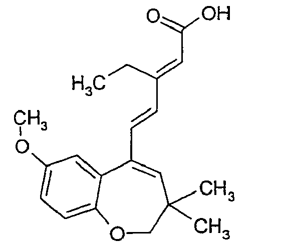 | (2E, 4E)-3-Ethyl-5-(7-methoxy-3,3-dimethyl-2,3-dihydrobenzo[b]-oxepin-5-yl)penta-2,4-dienoic acid |
| 44A | 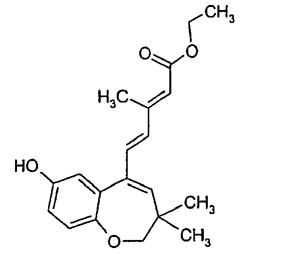 | (2E, 4E)-5-(7-Hydroxy-3,3-dimethyl-2,3-dihydrobenzo[b]-oxepin-5-yl)-3-methylpenta-2,4-dienoic acid ethyl ester |
| 44B | 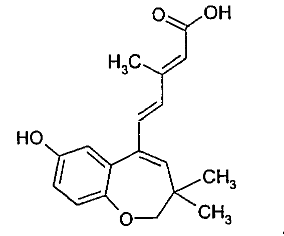 | (2E, 4E)-5-(7-Hydroxy-3,3-dimethyl-2, 3-dihydrobenzo[b]-oxepin-3-yl)-3-methylpenta-2,4-dienoic acid. |
| 45A | 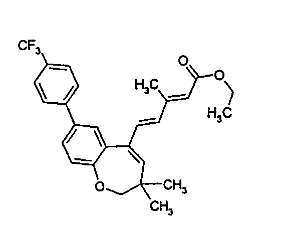 | (2E, 4E)-5-[3,3-Dimethyl-7-(4-(trifluoromethyl)phenyl)-2,3-dihydrobenzo[b]-oxepin-5-yl]-3-methylpenta-2,4-dienoic acid ethyl ester |
| 45B | 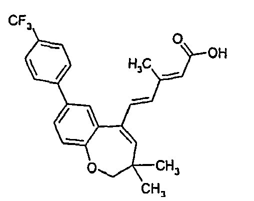 | (2E, 4E)-5-[3,3-Dimethyl-7-(4-(trifluoromethyl)phenyl)-2,3-dihydrobenzo [b] -oxepin-5-yl]-3-methylpenta-2,4-dienoic acid |
| 46A | 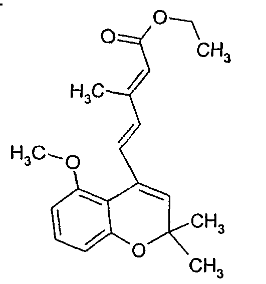 | (2E, 4E)-5-(5-Methoxy-2,2-dimethyl-2H-chromen-4-yl)-3-methylpenta-2,4-dienoic acid ethyl ester |
| 46B | 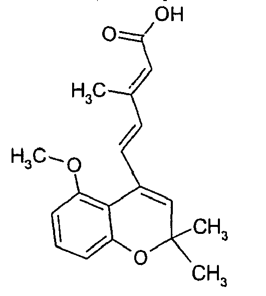 | (2E, 4E)-5-(5-Methoxy-2,2-dimethyl-2H-chromen-4-yl)-3-methylpenta-2,4-dienoic acid |
| 47A | 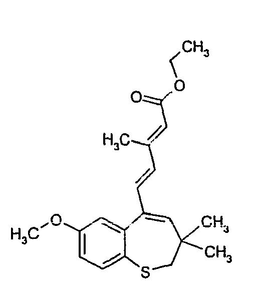 | (2E, 4E)-5-(7-Methoxy-3,3-dimethyl-2,3-dihydrobenzo[b]-thiepin-5-yl)-3-methylpenta-2,4-dienoic acid ethyl ester |
| 47B | 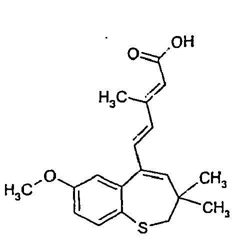 | (2E, 4E)-5-(7-Methoxy-3,3-dimethyl-2,3-dihydrobenzo[b]-thiepin-5-yl)-3-methylpenta-2,4-dienoic acid |
| 48A | 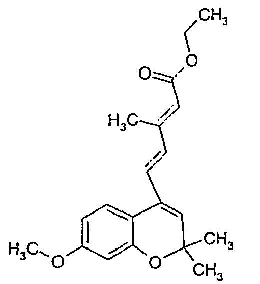 | (2E, 4E)-5-(7-Methoxy-2,2-dimethyl-2H-chromen-4-yl)-3-methylpenta-2,4-dienoic acid ethyl ester |
| 48B | 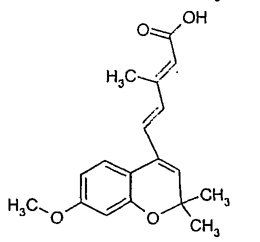 | (2E, 4E)-5-(7-Methoxy-2,2-dimethyl-2H-chromen-4-yl)-3-methylpenta-2,4-dienoic acid |
| 49A | 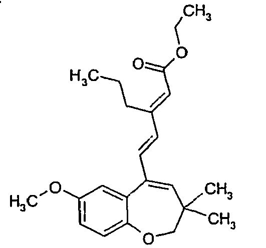 | (2E, 4E)-5-(7-Methoxy-3,3-dimethyl-2,3-dihydrobenzo[b]-oxepin-5-yl)-3-propylpenta-2,4-dienoic acid ethyl ester |
| 49B | 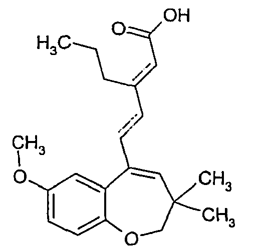 | (2E, 4E)-5-(7-Methoxy-3,3-dimethyl-2,3-dihydrobenzo[b]-oxepin-5-yl)-3-propylpenta-2,4-dienoic acid |
| 50A | 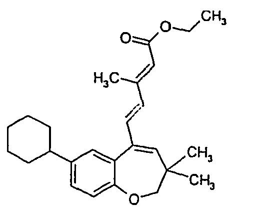 | (2E, 4E)-5-(7-Cyclohexyl-3,3-dimethyl-2,3-dihydrobenzo[b]-oxepin-5-yl)-3-methylpenta-2,4-dienoic acid ethyl ester |
| 50B | 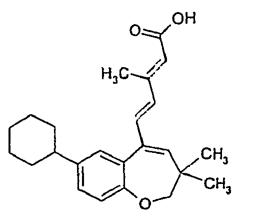 | (2E, 4E)-5-(7-Cyclohexyl-3,3-dimethyl-2,3-dihydrobenzo[b]-oxepin-5-yl)-3-methylpenta-2,4-dienoic acid |
| 51A | 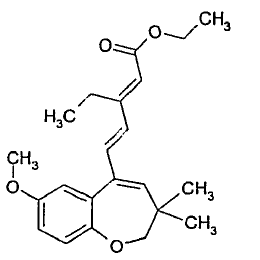 | (2Z, 4E)-3-Ethyl-5-(7-methoxy-3,3-dimethyl-2,3-dihydrobenzo[b]-oxepin-5-yl)penta-2,4-dienoic acid ethyl ester |
| 51B | 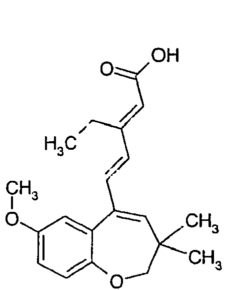 | (2Z, 4E)-3-Ethyl-5-(7-methoxy-3,3-dimethyl-2,3-dihydrobenzo [b]-oxepin-5-yl)penta2,4-dienoic acid |
| 52A | 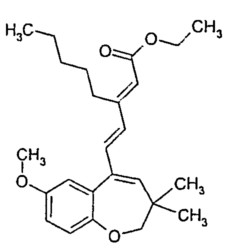 | (2E, 4E)-5-(7-Methoxy-3,3-dimethyl-2,3-dihydrobenzo[b]-oxepin-5-yl)-3-pentylpenta-2,4-dienoic acid ethyl ester |
| 52B | 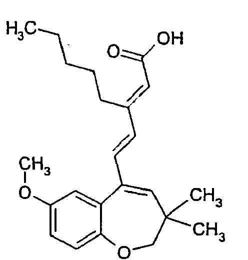 | (2E, 4E)-5-(7-Methoxy-3,3-dimethyl-2,3-dihydrobenzo[b]-oxepin-5-yl)-3-pentylpenta-2,4-dienoic acid |
| 53A | 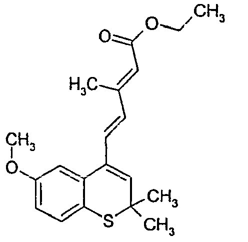 | (2E, 4E)-5-(2, 2-Dimethyl-thiochromen-4-yl)-3-methylpenta-2,4-dienoic acid ethyl ester |
| 53B | 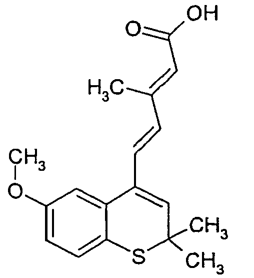 | (2E, 4E)-5-(2,2-Dimethylthio-chromene-4-yl)-3-methylpenta-2,4-dienoic acid |
| 54A | 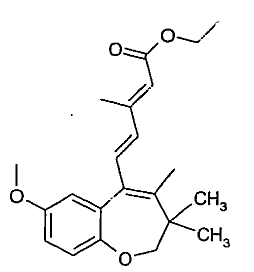 | (2E, 4E)-5-(7-Methoxy-3,3,4-trimethyl-2,3-dihydrobenzo[b]-oxepin-5-yl)-3-methylpenta-2,4-dienoic acid ethyl ester |
| 54B | 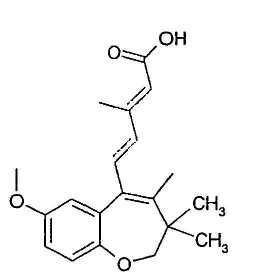 | (2E, 4E)-5-(7-Methoxy-3,3,4-trimethyl-2,3-dihydrobenzo[b]-oxepin-5-yl)-3-methylpenta-2,4-dienoic acid |
| 55A | 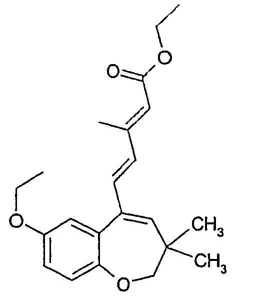 | (2E, 4E)-5-(7-Ethoxy-3,3-dimethyl-2,3-dihydrobenzo[b]- |
| 55B | 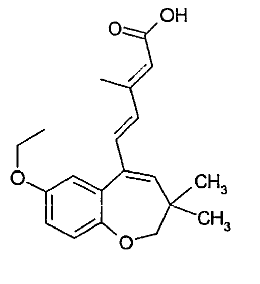 | (2E, 4E)-5-(7-Ethoxy-3,3-dimethyl-2,3-dihydrobenzo[b]-oxepin-5-yl)-3-methylpenta-2,4-dienoic acid |

The invention will now be described with reference to compound A.

### Experimental example 1

### Hypouricemic effect of compound A in healthy male volunteers

Forty-eight healthy male volunteers received orally either EMD or a placebo as a single administration in the morning. Six doses were administered 50 mg, 100 mg, 200 mg, 400 mg, 800 mg and 1200 mg once daily. In each group of dose, 6 subjects received compound A and 2 subjects received a placebo. Plasma uric acid concentrations were assessed in all dose groups.

In all subjects receiving compound A, a dose dependant decrease in plasma uric acid concentration was observed at 24 hours in all groups of dose. The drop of plasma uric acid concentration was around 45 % for the lowest tested dose (50 mg) and the maximum effect was observed from the 800 mg dose (80 %). No change was observed in placebo.

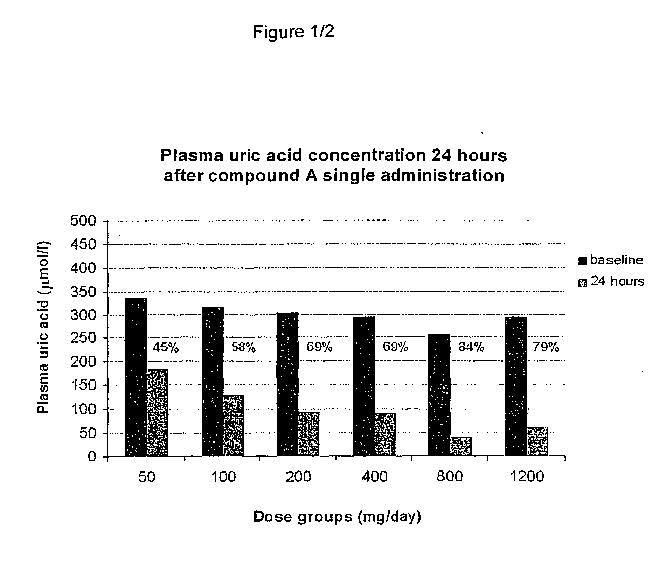

- Figure 1:: Mean plasma uric acid concentration in each groupof dose before and 24 hours after drug intake (single dose) in subjects receiving compound A.
Experimental example 2
Sixteen healthy male volunteers received orally either EMD or a placebo as a single administration in the morning (Day 1) followed 3 days later by a repeated administration during 7 days (Day 4 to Day 10); 100 mg and 200 mg were administered once daily. In each group of dose, 6 subjects received compound A and 2 subjects received a placebo. No change was observed in placebo.

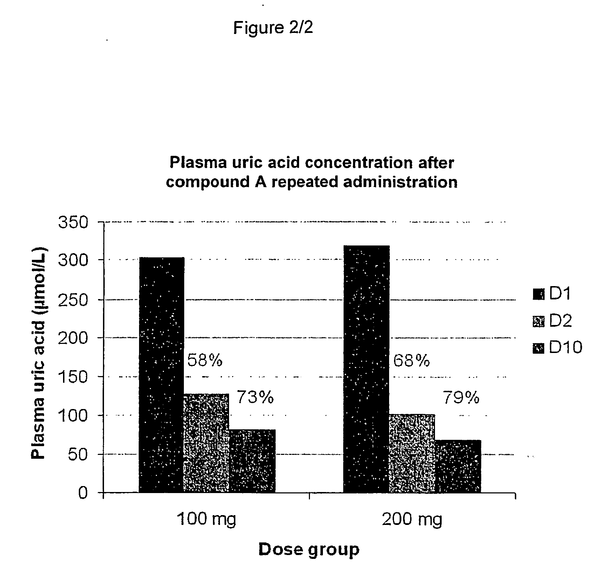

- Figure 2 :: Mean plasma uric acid concentration per dose group as a function of time and drug dose in subjects receiving repeated administration of compound A.

### REFERENCES

■ Oxford Textbook of Clinical Nephrology Second Edition. Edited by Davison A.M., Cameron J.S., Grünfeld J.P., Kerr D.N.S., Ritz E. and Winearls C.G. Oxford University Press*.*
   - 6. The patient with chronic interstitial disease 6.4 Uric acid and the kidney. Cameron S.J., Moro F. and Simmonds H.A. 1157-1173
   - 8. The patient with renal stone disease
      8.1 Aetiological factors in stone formation. Watts R.W.E.,
         Uric acid and urate stones, 1333-1334
      8.2 The medical management of stone disease. Sutton R.A.L.
         Uric acid stones, 1352-1353
   - 19.2 Handling of drugs in kidney disease. Carmichael D.J.S.
      Hyperuricemia, anti-inflammatory agents, 2671-2672
■ The Kidney, Brenner & Rector's Fifth Edition. Edited by Brenner B.M. W.B. Saunders Company*.*
   - 15. Renal hanling of organic anions and cations and renal excretion on uric acid. Sica D.A., Schoolwerth A.C.
      Urate transport 613-617
   - 28. Acute renal failure. Brady H.R., Brenner B.M., Lieberthal W.
      Acute tubule necrosis. 1204-1207
      Intrinsic renal azotemia, prevention. 1232
   - 31. Secondary glomerular diseases. Adler S.G., Cohen A.H., Glassock R. J.
      Medications, immunizations, and allergens. 1563-1566
   - 33. Tubulointerstitial Diseases. Kelly C.J., Neilson E.G.
      Acute interstitial nephritis. 1661-1665
      Chronic interstitial nephritis, uric acid nephropathy. 1669
   - 34. Toxic Nephropathy. Cronin R., Henrich W.L,
      Nephrotoxicity of tumor cell lysis 1692
   - 40. Nephrolithiasis. Asplin J.R., Favus M.J., Coe F.L.
      Hyperuricosuria 1912-1915
      Uric acid stones. 1922-1924
■ Renal Pathology with Clinical and Functional Correlations, 2nd Edition Edited by Tisher C. C., Brenner B. J.B. Lippincott company
   - 45. Urate and Uric Acid Nephropathy, Cystinosis, and Oxalosis. Chonko A.M., Richardson W.P.
      Urate and uric acid nephropathy. 1413-1423
■ Rheumatology, Edited by Klippel J.H., Dieppe P.A., Brooks p, Carette S., Dequeker J., Gerber L.H., Hazleman B.L., Keat A.C.S., Kimberly R.P., Liang M.H., Maini R.N., van de Putte L., Sturrock R.D., Urowitz M.B., Wollheim F.A., Kimberly R.P., Zvaifler N.J. Mosby*.*
   Part 7: Disorders of Bone, Cartilage and Connective Tissue. Dequeker J., van de Putte L.
   - 12. Crystal arthropathies: Gout. Cohen M. G. & Emmerson B.T. 12.1-12.16
   Part 8: Management of Rheumatic Diseases. Brooks P.M. & Gerber L.H
   - Pharmacologic approaches, NSAIDs. Brooks P., 10.1-10.6
   - Pharmacologic approaches, Systemic corticosteroids in rheumatology. Kirwan J.R. 11.1-11.6
   - Pharmacological approaches, Antihyperuricemics. Emmerson B.T. 15.1-15.5
■ Principles of Internal Medicine, Harrison's 14 th Edition edited by Fauci A.S., Martin J.B., Braunwald E., Kasper D.L., Isselbacher K.J., Hauser S. L., Wilson J.D., Longo D.L. McGraw-Hill
   Part Ten: Disorders of the Kidney and Urinary Tract
   - 270: Acute renal failure. Brady H.R., Brenner B.M. 1504-1513
   - 271: Chronic renal failure. Lazarus J.M., Brenner B.M. 1513-1520
   - 276: Tubulointerstitial diseases of the kidney. Brenner B.M., Levy E., Hostetter T.H. 1553-1556
   Part Thirteen: Endocrinology and Metabolism
   - Section 2: Disorders of Intermediary Metabolism 344: Gout and other disorders of purine metabolism. Wortmann R.L. 2158-2166
■ The Pharmacological Basis of Therapeutics, Goodman & Gilman's, Ninth Edition, Edited by Hardman J.G., Limbird L.E., Molinoff P.B., Ruddon R.W., Goodman Gilman A. McGraw-Hill
   Section IV, Autacoids; Drug Therapy of Inflammation
   - 27. Analgesic-antipyretic and antiinflammatory agents and drugs employed in the treatment of gout. Insel P.A.
      Drug employed in the treatment of gout 647-650;
      Uricosuric agents 650-653
      Treatment of gout and hyperuricemia 653-655
■ Terkeltaub R.A. Gout : Clinical Practice. N Engl J. Med 2003; 349:1647-55
■ Watanabe S, Kang D-H, Feng L, Nakagawa T, Kanellis J, Lan H, Mazzali M, Johnson R. J. Uric Acid, Hominoid Evolution, and the Pathogenesis of Salt-Sensitivity. Hypertension 2002; 40: 355-360

## Claims

1. The use of a pentadienoic acid derivative of formula (I) for the preparation of a medicament for the prevention or treatment of hyperuricemia and/or one or several associated disorders or diseases, and/or for reducing the serum uric acid level of a subject. 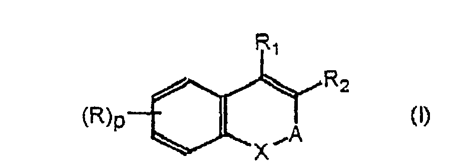 in which:
X represents O or S;
A represents either the divalent radical - (CH₂) _{S}-CO- (CH₂) ₜ- or the divalent radical - (CH₂)ₛ-CR₃R₄-(CH₂) t-
in which radicals s = t = 0 or else one of s and t has the value 0 and the other has the value 1;
R₄ represents a hydrogen atom or a (C₁-C₁₅)alkyl group;
R₁ and R₂ independently represent the Z chain defined below; a hydrogen atom; a (C₁-C₁₈)alkyl group; a (C₂-C₁₈)alkenyl group; a (C₂-C₁₈)alkynyl group; a (C₆-C₁₀)aryl group optionally substituted by a halogen atom, by an optionally halogenated (C₁-C₅)alkyl group or by an optionally halogenated (C₁-C₅)alkoxy group; or a mono- or bicyclic (C₄-C₁₂)heteroaryl group comprising one or more heteroatoms chosen from O, N and S which is optionally substituted by a halogen atom, by an optionally halogenated (C₁-C₅)alkyl group or by an optionally halogenated (C₁-C₅) alkoxy group;
R₃ and R₄ independently takes any one of the meanings given above for R₁ and R₂, with the exception of the Z chain; or else
R₃ and R₄ together form a (C₂-C₆) alkylene chain optionally substituted by a halogen atom or by optionally halogenated (C₁-C₅)alkoxy;
R is chosen from a halogen atom; a cyano group; a nitro group; a carboxy group; an optionally halogenated (C₁-C₁₈)alkoxycarbonyl group; an Rₐ-CO-NH- or RₐR_{b}N-CO- group [in which Rₐ and R_{b} independently represent optionally halogenated (C₁-C₁₈)alkyl; a hydrogen atom; (C₆-C₁₀)aryl or (C₆-C₁₀)aryl(C₁-C₅)alkyl (where the aryl parts are optionally substituted by a halogen atom, by an optionally halogenated (C₁-C₅) alkyl group or by an optionally halogenated (C₁-C₅) alkoxy group) ; (C₃-C₁₂) cycloalkyl optionally substituted by a halogen atom, by an optionally halogenated C₁-C₅ alkyl [sic] group or by an optionally halogenated (C₁-C₅)alkoxy group]; an optionally halogenated (C₁-C₁₈) alkyl group; optionally halogenated (C₁-C₁₈) alkoxy; and (C₆-C₁₀) aryl, (C₆-C₁₀) aryl (C₁-C₅) alkyl, (C₆-C₁₀) aryloxy, (C₃-C₁₂) cycloalkyl, (C₃-C₁₂) cycloalkenyl, (C₃ - C₁₂) cycloalkyloxy or (C₃-C₁₂) cycloalkenyloxy in which the aryl, cycloalkyl and cycloalkenyl parts are optionally substituted by a halogen atom, by optionally halogenated (C₁-C₅) alkyl or by optionally halogenated (C₁-C₅) alkoxy;
-OH;
p represents 0, 1, 2, 3 or 4;
Z represents the radical: 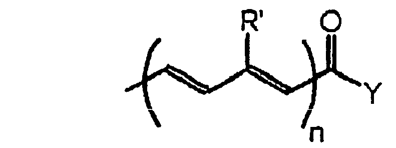 where n is 1 or 2;
the R' groups independently represent a hydrogen atom; a (C₁-C₅) alkyl group; a (C₆-C₁₀) aryl group optionally substituted by a halogen atom, by an optionally halogenated (C₁-C₅)alkyl group or by optionally halogenated (C₁-C₅)alkoxy; or a mono- or bicyclic (C₄-C₁₂)heteroaryl group comprising one or more heteroatoms chosen from O, N and S which is optionally substituted by a halogen atom, by an optionally halogenated (C₁-C₅)alkyl group or by an optionally halogenated (C₁-C₅)alkoxy group;
Y represents -OH; (C₁-C₅) alkoxy; or the -NR_{c}R_{d} group (in which R_{c} and R_{d} independently represent a hydrogen atom; (C₁-C₅) alkyl; (C₃-C₈) cycloalkyl optionally substituted by a halogen atom, by optionally halogenated (C₁-C₅)alkyl or by optionally halogenated (C₁-C₅)alkoxy; (C₆-C₁₀) aryl optionally substituted by a halogen atom, by optionally halogenated (C₁-C₅) alkyl or by optionally halogenated (C₁-C₅) alkoxy;
Or Y represents glucomic acid 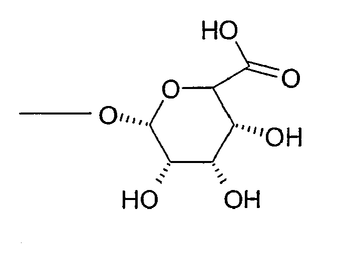 it being understood that one and one alone from R₁ and R₂ represents the Z chain;
and their pharmaceutically acceptable salts with acids or bases, or esters.

2. The use according to Claim 1, **characterized in that** A represents the divalent radical - (CH₂)ₛ-CR₃R₄-(CH₂)ₜ- in which s, t, R₃ and R₄ are as defined in Claim 1.

3. The use according to Claim 1, **characterized in that**:
X represents 0;
A represents -CR₃R₄- or -CH₂-CR₃R₄- in which the unsubstituted methylene group is bonded to X;
R₁ and R₂ independently represent Z; H; (C₁-C₁₅)alkyl; (C₂-C₁₅)alkenyl; or phenyl optionally substituted by (C₁-C₅) alkyl, (C₁-C₅)alkoxy, a halogen atom or -CF₃;
R₃ and R₄ independently takes any one of the meanings given above for R₁ and R₂, with the exception of Z;
R is chosen from (C₁-C₉)alkyl; (C₁-C₅)alkoxy; phenyl or phenylcarbonyl optionally substituted by a halogen atom, (C₁-C₅) alkyl, (C₁-C₅)alkoxy, -CF₃ or -OCF₃; a halogen atom; - CF₃ and -OCF₃;
Z represents the radical: 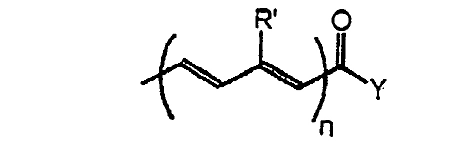
where n represents 1; and
R' represents (C₁-C₅) alkyl or (C₆-C₁₀) aryl.

4. The use according to any one of Claims 1 to 3, wherein :
X represents O;
A represents -CH₂-CR₃R₄- in which the unsubstituted methylene group is bonded to X;
R₁ and R₂ independently represent Z, a hydrogen atom or (C₁-C₅) alkyl;
R₃ and R₄ independently takes any one of the meanings given above for R₁ and R₂, with the exception of Z;
Z represents 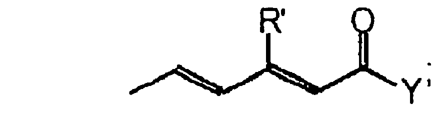 and
R' represents methyl or phenyl.

5. The use according to anyone of claims 1 to 4, wherein R1 represents Z.

6. The use according to anyone of claims 1 to 5, wherein R₂ represents a hydrogen atom.

7. The use according to anyone of claims 1 to 6, wherein Y is a (C₁-C₅) alkoxy.

8. The use according to any one of Claims 1 to 6, wherein :
Y represents -OH; (C₁-C₅)alkoxy; or -NR_{c}R_{d} in which R_{c} and R_{d} are as defined in Claim 1.

9. The use according to anyone of claims 1 to 8, wherein R' is methyl.

10. The use according to any of claims 1 to 9, wherein R is (C₁-C₅) alkoxy.

11. The use according to any one of Claims 1 to 6, wherein p represents 0, 1 or 2.

12. The use according to claim 1, wherein :
[X represents O ;
A represents -CH₂-CR₃R₄- in which the unsubstituted methylene group is bonded to X ;
R₁ is Z and R₂ is H;
R₃ and R₄ independently represents a (C₁-C₅) alkyl group;
R is a (C₁-C₅) alkoxy;
Z represents 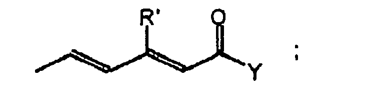
wherein R' represents a methyl or phenyl ; and y represents a (C₁-C₅) alkoxy] .

13. The use according to claim 1 wherein said derivative is selected from the group consisting of
- (2E, 4E)-5-(2-pentyl-2H-1-benzopyran-3-yl)-3-methylpenta-2,4-dienoic acid;
- (2Z, 4E)-5-(2-pentyl-2H-1-benzopyran-3-yl)-3-methylpenta-2,4-dienoic acid;
- (2E, 4E)-5-(2,2-dimethyl-6-methoxy-2H-1-benzopyran-3-yl)-3-methylpenta-2,4-dienoic acid;
- (2E, 4E)-5-(2H-1-benzopyran-3-yl)-3-methylpenta-2,4-dienoic acid;
- (2E, 4E)-5-(2,2-dimethyl-2H-1-benzopyran-3-yl)-3-methylpenta-2,4-dienoic acid;
- (2Z, 4E)-5-(2,2-dimethyl-2H-1-benzopyran-3-yl)-3-methylpenta-2,4-dienoic acid;
- (2E, 4E)-5-[2-(non-6-enyl)-2H-1-benzopyran-3-yl]-3-methylpenta-2,4-dienoic acid;
- (2E, 4E)-5-(4-phenyl-2H-1-benzopyran-3-yl)-3-methylpenta-2,4-dienoic acid;
- (2E, 4E)-5-(6-nonyl-2H-1-benzopyran-3-yl)-3-methylpenta-2,4-dienoic acid;
- (2E, 4E)-5-(6-phenyl-2H-1-benzopyran-3-yl)-3-methylpenta-2,4-dienoic acid;
- (2E, 4E)-5-(2-nonyl-2H-1-benzopyran-3-yl)-3-methylpenta-2,4-dienoic acid;
- (2E, 4E)-5-(4-methyl-2H-1-benzopyran-3-yl)-3-methylpenta-2,4-dienoic acid;
- (2Z, 4E)-5-(2H-1-benzopyran-3-yl)-3-methylpenta-2,4-dienoic acid;
- (2E, 4E)-5-(2-undecanyl-2H-1-benzopyran-3-yl)-3-methylpenta-2,4-dienoic acid;
- (2E, 4E)-5-(2-phenyl-2H-1-benzopyran-3-yl)-3-methylpenta-2,4-dienoic acid;
- (2E, 4E)-5-(5-methyl-2,3-dihydrobenzoxepin-4-yl)-3-methylpenta-2,4-dienoic acid;
- (2E, 4E)-5-(3,3-dimethyl-7-methoxy-2,3-dihydrobenzoxepin-5-yl)-3-methylpenta-2,4-dienoic acid; and
- (2E, 4E)-5-(2,3-dihydrobenzoxepin-4-yl)-3-methylpenta-2,4-dienoic acid;
- (2E, 4E)-5-(3,3-dimethyl-7-methoxy-2,3-dihydrobenzoxepin-5-yl)-3-phenylpenta-2,4-dienoic acid;
- (2Z, 4E)-5-(3,3-dimethyl-7-methoxy-2,3-dihydrobenzoxepin-5-yl)-3-phenylpenta-2,4-dienoic acid;
- (2Z, 4E)-5-(3,3-dimethyl-7-methoxy-2,3-dihydrobenzoxepin-5-yl)-3-methylpenta-2,4-dienoic acid;
- (2E, 4E)-5-(3,3-dimethyl-7,8-dimethoxy-2,3-dihydrobenzoxepin-5-yl)-3-methylpenta-2,4-dienoic acid;
- (2E, 4E)-5-(3,3-dimethyl-2,3-dihydrobenzoxepin-5-yl)-3-methylpenta-2,4-dienoic acid;
- (2E, 4E)-5-(3,3-dimethyl-2,3-dihydro-7-(para-chlorobenzoyl)benzoxepin-5-yl)-3-methylpenta-2,4-dienoic acid;
- (2E, 4E)-5-(3,3-dimethyl-7-chloro-2,3-dihydrobenzoxepin-5-yl)-3-methylpenta-2,4-dienoic acid;
- (2E, 4E)-5-(3,3-dimethyl-7,8-dichloro-2,3-dihydrobenzoxepin-5-yl)-3-methylpenta-2,4-dienoic acid;
- (2E, 4E)-5-(3,3-dimethyl-7-bromo-2,3-dihydrobenzoxepin-5-yl)-3-methylpenta-2,4-dienoic acid;
- (2E, 4E)-5-(3,3-dimethyl-7-fluoro-8-chloro-2,3-dihydrobenzoxepin-5-yl)-3-methylpenta-2,4-dienoic acid;
- (2E, 4E)-5-(3,3-dimethyl-7-fluoro-2,3-dihydrobenzoxepin-5-yl)-3-methylpenta-2,4-dienoic acid;
- (2E, 4E)-5-(3,3-dimethyl-7-trifluoromethyl-2,3-dihydrobenzoxepin-5-yl)-3-methylpenta-2,4-dienoic acid;
- (2E, 4E)-5-(3,3-dimethyl-7-phenyl-2,3-dihydrobenzoxepin-5-yl)-3-methylpenta-2,4-dienoic acid;
- (2E, 4E)-5-(3,3,7-trimethyl-2,3-dihydrobenzoxepin-5-yl)-3-methylpenta-2,4-dienoic acid;
- (2E, 4E)-5-(3,3-dimethyl-2,3-dihydrobenzoxepin-5-yl)-3-methylpenta-2,4-dienoic acid;
- (2E, 4E)-5-(9-methoxy-3,3-dimethyl-2,3-dihydrobenzoxepin-5-yl)-3-methylpenta-2,4-dienoic acid;
and their pharmaceutically acceptable esters.

14. The use according to anyone of claims 1 to 13 wherein the diseases associated with hyperuricemia to be treated comprise one or several of the following : gout, acute inflammatory arthritis, tophaceous deposition of urate crystals in and around joints, chronic arthritis, deposition of urate crystals in renal parenchyma, urolithiasis, and related renal disease also termed gouty nephropaty.

15. The use according to anyone of claims 1 to 13 wherein the hyperuricemiae to be treated comprises primary and secondary hyperuricemiae, such as drug related to hyperuricemiae (e.g. by diuretics, immunosuppressive of cytotoxic agents), or hyperuricemiae related to diverse medical conditions (e.g. nephropaties, myeloproliferative disorders, conditions associated with insuline resistance and transplantations).

16. The use according to any one of claims 1 to 13 to prepare medicaments for subjects having serum uric acid levels, before treatment, equal or above 7 mg/dL (420 %m/L) .

17. The use according to claim 16 where the conditions to be treated are gout or any condition brought about by high levels of uric acid in the joints or kidneys or a serum level over 9 mg/dL (530µ mol/L).

18. The use according to any of claims 1 to 17 for preparing a medicament suitable for administering the 2,4-pentadienoic acid derivative of formula (I) by the oral route.

19. The use according to one of claims 1 to 18 for preparing a medicament for administering the effective amount of 2,4-pentadienoic acid or derivative according to formula (I) once or twice per day.

20. The use according to any one of claims 1 to 19, wherein the amount of said pentadienoic acid derivative is substantially lower than the amount needed for the relevant derivative as used in the treatment of dyslipidemia, atherosclerosis and diabetes.

21. The use according to claim 20 wherein said amount is at least 50% lower.

22. The use according to claim 21 wherein said amount is at least 90% lower.

23. The use according to any one of claims 1 to 20, wherein the amount of said pentadienoic acid derivative is from 0.15 to 4 mg/Kg of human body weight.

24. The use according to claim 23, wherein said amount is from 0.3 to 1.0 mg/Kg human body weight.

25. The use according to one of claims 1 to 24 wherein said derivative is (2E,4E)-5-(3,3-dimethyl-7-methoxy-2,3-dihydrobenzo-xepin-5-yl)-3-methylpenta-2,4-dienoic acid, or its pharmaceutically acceptable salts or esters, among which its ethyl ester.

26. New medical compositions which comprise, in a vehicle acceptable for a human, an effective amount of at least one 2,4-pentadienoic acid derivative as defined in anyone of claims 1 to 13,
wherein the effective amount in a dose for a one day administration for an adult is from 0.15 to 4 mg/kg of a human body.

27. Medical compositions according to claim 26, wherein said effective amount is from 0.3 to 1.0 mg/Kg of a human body.

28. Medical compositions according to claim 26 or 27 formulated for oral administration.

29. A medicament according to anyone of claims 26 to 28 wherein said derivative is (2E,4E)-5-(3,3-dimethyl-7-methoxy-2,3-dihydrobenzo-xepin-5-yl)-3-methylpenta-2,4-dienoic acid, or its pharmaceutically acceptable salts or esters, among which its ethyl ester.

## Patentansprüche

1. Verwendung eines Pentadiensäurederivats der Formel (I) zur Herstellung eines Arzneimittels zur Vorbeugung oder Behandlung von Hyperurikämie und/oder einer oder mehreren damit einhergehenden Störungen oder Erkrankungen und/oder zur Verminderung des Serumspiegels an Harnsäure eines Subjekts, 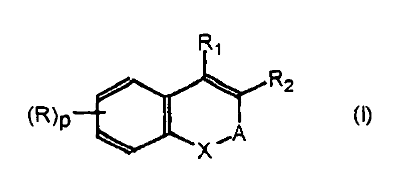 in der:
X O oder S bedeutet;
A entweder den zweiwertigen Rest -(CH₂)ₛ-CO-(CH_{z})ₜ- oder den zweiwertigen Rest - (CH₂)ₛ-CR₃R₄-(CH₂)ₜ- bedeutet
wobei in diesen Resten s = t = 0 oder auch einer von s und t den Wert 0 aufweist und der andere den Wert 1 aufweist;
R₄ ein Wasserstoffatom oder eine (C₁-C₁₅)-Alkylgruppe bedeutet;
R₁ und R₂ unabhängig voneinander die unten definierte Z-Kette; ein Wasserstoffatom; eine (C₁-C₁₈)-Alkylgruppe; eine (C₂-C₁₈) -Alkenylgruppe; eine (C₂-C₁₈) -Alkinylgruppe; eine (C₆-C₁₀) -Arylgruppe, gegebenenfalls substituiert durch ein Halogenatom, durch eine gegebenenfalls halogenierte (C₁-C₅)-Alkylgruppe oder durch eine gegebenenfalls halogenierte (C₁-C₅)-Alkoxygruppe; oder eine mono- oder bicyclische (C₄-C₁₂) -Heteroarylgruppe, die ein oder mehrere Heteroatome ausgewählt aus O, N und S enthält und die gegebenenfalls durch ein Halogenatom, durch eine gegebenenfalls halogenierte (C₁-C₅)-Alkylgruppe oder durch eine gegebenenfalls halogenierte (C₁-C₅) -Alkoxygruppe substituiert ist, bedeuten;
R₃ und R₄ unabhängig voneinander eine der oben für R₁ und R₂ angegebenen Bedeutungen annehmen, mit Ausnahme der Z-Kette; oder auch
R₃ und R₄ zusammen eine (C₂-C₆)-Alkylenkette bilden, die gegebenenfalls durch ein Halogenatom oder durch gegebenenfalls halogeniertes (C₁-C₅)-Alkoxy substituiert ist;
R aus einem Halogenatom; einer Cyanogruppe; einer Nitrogruppe; einer Carboxygruppe; einer gegebenenfalls halogenierten (C₁-C₁₈)-Alkoxycarbonylgruppe; einer Rₐ-CO-NH- oder RₐR_{b}N-CO-Gruppe [in der Rₐ und R_{b} unabhängig voneinander gegebenenfalls halogeniertes (C₁-C₁₈)-Alkyl; ein Wasserstoffatom; (C₆-C₁₀) -Aryl oder (C₆-C₁₀) -Aryl- (C₁-C₅) -Alkyl (wobei die Arylteile gegebenenfalls durch ein Halogenatom, durch eine gegebenenfalls halogeniert (C₁-C₅) -Alkylgruppe oder durch eine gegebenenfalls halogenierte (C₁-C₅)-Alkoxygruppe substituiert sind); (C₃-C₁₂) -Cycloalkyl, gegebenenfalls substituiert durch ein Halogenatom, durch eien gegebenenfalls halogenierte C₁-C₅-Alkylgruppe oder durch eine gegebenenfalls halogenierte (C₁-C₅)-Alkoxygruppe bedeuten] ; einer gegebenenfalls halogenierten (C₁-C₁₈) -Alkylgruppe; gegebenenfalls halogenierten (C₁-C₁₈)-Alkoxy; und (C₆-C₁₀) -Aryl, (C₆-C₁₀) -Aryl- (C₁-C₅) -Alkyl, (C₆-C₁₀) -Aryloxy, (C₃-C₁₂) -Cycloalkyl, (C₃-C₁₂) -Cycloalkenyl, (C₃-C₁₂) -Cycloalkyloxy oder (C₃-C₁₂)-Cycloalkenyloxy, in denen die Aryl-, Cycloalkyl- und Cycloalkenylteile gegebenenfalls substituiert sind durch ein Halogenatom, durch gegebenenfalls halogeniertes (C₁-C₅)-Alkyl oder durch gegebenenfalls halogeniertes (C₁-C₅)-Alkoxy; -OH; ausgewählt wird
p 0, 1, 2, 3 oder 4 bedeutet;
Z den Rest: 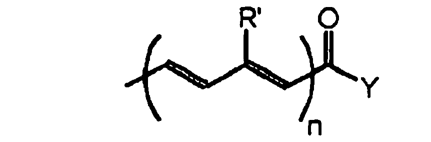 bedeutet, wobei n 1 oder 2 ist;
die R'-Gruppen unabhängig voneinander ein Wasserstoffatom; eine (C₁-C₅) -Alkylgruppe; eine (C₆-C₁₀) -Arylgruppe, gegebenenfalls substituiert durch ein Halogenatom, durch eine gegebenenfalls halogenierte (C₁-C₅)-Alkylgruppe oder durch eine gegebenenfalls halogenierte (C₁-C₅)-Alkoxy; oder eine mono- oder bicyclische (C₄-C₁₂)-Heteroarylgruppe, die ein oder mehrere Heteroatome enhält, ausgewählt aus O, N und S, und die gegebenenfalls substituiert ist durch ein Halogenatom, durch eine gegebenenfalls halogenierte (C₁-C₅) - Alkylgruppe oder durch eine gegebenenfalls halogenierte (C₁-C₅)-Alkoxygruppe, bedeuten;
Y -OH; (C₁-C₅) -Alkoxy; oder die -NR_{c}R_{d}-Gruppe (in der R_{c} und R_{d} unabhängig voneinander ein Wasserstoffatom; (C₁-C₅)-Alkyl; (C₃-C₈)-Cycloalkyl, gegebenenfalls substituiert durch ein Halogenatom, durch gegebenenfalls halogeniertes (C₁-C₅)-Alkyl oder durch gegebenenfalls halogeniertes (C₁-C₅)-Alkoxy; (C₆-C₁₀) -Aryl, gegebenenfalls substituiert durch ein Halogenatom, durch gegebenenfalls halogeniertes (C₁-C₅)-Alkyl oder durch gegebenenfalls halogeniertes (C₁-C₅)-Alkoxy, bedeuten) bedeutet;
oder Y Glucuronsäure 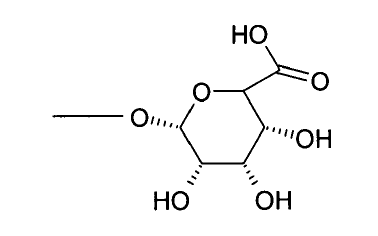 bedeutet, wobei es sich versteht, dass eines und nur eines aus R₁ und R₂ die Z-Kette bedeutet;
und ihrer pharmazeutisch unbedenklichen Salze mit Säuren oder Basen, oder Ester.

2. Verwendung nach Anspruch 1, **dadurch gekennzeichnet, dass** A den zweiwertigen Rest -(CH₂)ₛ-CR₃R₄-(CH₂)ₜ- bedeutet, in dem s, t, R₃ und R₄ die in Anspruch 1 angegebenen Bedeutungen haben.

3. Verwendung nach Anspruch 1, **dadurch gekennzeichnet, dass**:
X O bedeutet;
A -CR₃R₄- oder -CH₂-CR₃R₄- bedeutet, in denen die unsubstituierte Methylengruppe an X gebunden ist;
R₁ und R₂ unabhängig voneinander Z; H; (C₁-C₁₅)-Alkyl; (C₂-C₁₅)-Alkenyl; oder gegebenenfalls durch (C₁-C₅)-Alkyl, (C₁-C₅)-Alkoxy, ein Halogenatom oder -CF₃ substituiertes Phenyl bedeuten;
R₃ und R₄ unabhängig voneinander eine beliebige der oben für R₁ und R₂ angegebenen Bedeutungen annehmen, mit Ausnahme von Z;
R aus (C₁-C₉) -Alkyl; (C₁-C₅)-Alkoxy; Phenyl oder Phenylcarbonyl, gegebenenfalls substituiert durch ein Halogenatom, (C₁-C₅)-Alkyl, (C₁-C₅)-Alkoxy, -CF₃ oder -OCF₃; einem Halogenatom; -CF₃ und -OCF₃ ausgewählt wird;
Z den Rest: 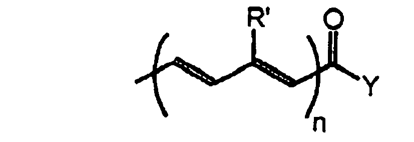 bedeutet, wobei n 1 bedeutet; und
R' (C₁-C₅) -Alkyl oder (C₆-C₁₀) -Aryl bedeutet.

4. Verwendung nach einem der Ansprüche 1 bis 3, wobei:
X O bedeutet;
A -CH₂-CR₃R₄- bedeutet, in dem die unsubstituierte Methylengruppe an X gebunden ist;
R₁ und R₂ unabhängig voneinander Z, ein Wasserstoffatom oder (C₁-C₅)-Alkyl bedeuten;
R₃ und R₄ unabhängig voneinander eine beliebe der oben für R₁ und R₂ angegebenen Bedeutungen annehmen, mit Ausnahme von Z;
Z bedeutet 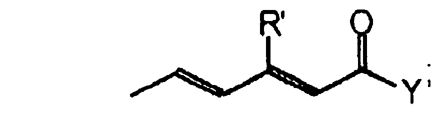 und
R' Methyl oder Phenyl bedeutet.

5. Verwendung nach einem der Ansprüche 1 bis 4, wobei R₁ Z bedeutet.

6. Verwendung nach einem der Ansprüche 1 bis 5, wobei R₂ ein Wasserstoffatom bedeutet.

7. Verwendung nach einem der Ansprüche 1 bis 6, wobei Y ein (C₁-C₅) -Alkoxy ist.

8. Verwendung nach einem der Ansprüche 1 bis 6, wobei:
Y -OH; (C₁-C₅)-Alkoxy; oder -NR_{c}R_{d} bedeutet, in dem R_{c} und R_{d} die in Anspruch 1 angegebenen Bedeutungen haben.

9. Verwendung nach einem der Ansprüche 1 bis 8, wobei R' Methyl ist.

10. Verwendung nach einem der Ansprüche 1 bis 9, wobei R (C₁-C₅) -Alkoxy ist.

11. Verwendung nach einem der Ansprüche 1 bis 6, wobei P 0, 1 oder 2 bedeutet.

12. Verwendung nach Anspruch 1, wobei:
[X O bedeutet;
A CH₂-CR₃R₄- bedeutet, in dem die unsubstituierte Methylengruppe an X gebunden ist;
R₁ Z ist und R₂ H ist;
R₃ und R₄ unabhängig voneinander eine (C₁-C₅) -Alkylgruppe bedeuten;
R ein (C₁-C₅)-Alkoxy ist;
Z 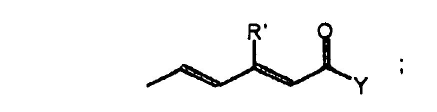
bedeutet, wobei R' ein Methyl oder Phenyl bedeutet; und y ein (C₁-C₅)-Alkoxy bedeutet] .

13. Verwendung nach Anspruch 1, wobei dieses Derivat ausgewählt wird aus der Gruppe bestehend aus
- (2E,4E)-5-(2-Pentyl-2H-1-benzopyran-3-yl)-3-methylpenta-2,4-diensäure;
- (2Z,4E)-5-(2-Pentyl-2H-1-benzopyran-3-yl)-3-methylpenta-2,4-diensäure;
- (2E,4E)-5-(2,2-Dimethyl-6-methoxy-2H-1-benzopyran-3-yl)-3-methylpenta-2,4-diensäure;
- (2E,4E)-5-(2H-1-Benzopyran-3-yl)-3-methylpenta-2,4-diensäure;
- (2E,4E)-5-(2,2-Dimethyl-2H-1-benzopyran-3-yl)-3-methylpenta-2,4-diensäure;
- (2Z,4E)-5-(2,2-Dimethyl-2H-1-benzopyran-3-yl)-3-methylpenta-2,4-diensäure;
- (2E,4E)-5-[2-(Non-6-enyl)-2H-1-benzopyran-3-yl]-3-methylpenta-2,4-diensäure;
- (2E,4E)-5-(4-Phenyl-2H-1-benzopyran-3-yl)-3-methylpenta-2,4-diensäure;
- (2E,4E)-5-(6-Nonyl-2H-1-benzopyran-3-yl)-3-methylpenta-2,4-diensäure;
- (2E,4E)-5-(6-Phenyl-2H-1-benzopyran-3-yl)-3-methylpenta-2,4-diensäure;
- (2E,4E)-5-(2-Nonyl-2H-1-benzopyran-3-yl)-3-methylpenta-2,4-diensäure;
- (2E,4E)-5-(4-Methyl-2H-1-benzopyran-3-yl)-3-methylpenta-2,4-diensäure;
- (2Z,4E)-5-(2H-1-Benzopyran-3-yl)-3-methylpenta-2,4-diensäure;
- (2E,4E)-5-(2-Undecanyl-2H-1-benzopyran-3-yl)-3-methylpenta-2,4-diensäure;
- (2E,4E)-5-(2-Phenyl-2H-1-benzopyran-3-yl)-3-methylpenta-2,4-diensäure;
- (2E,4E)-5-(5-Methyl-2,3-dihydrobenzoxepin-4-yl)-3-methylpenta-2,4-diensäure;
- (2E,4E)-5-(3,3-Dimethyl-7-methoxy-2,3-dihydrobenzoxepin-5-yl)-3-methylpenta-2,4-diensäure;
- (2E,4E)-5-(2,3-Dihydrobenzoxepin-4-yl)-3-methylpenta-2,4-diensäure;
- (2E,4E)-5-(3,3-Dimethyl-7-methoxy-2,3-dihydrobenzoxepin-5-yl)-3-methylpenta-2,4-diensäure;
- (2Z,4E)-5-(3,3-Dimethyl-7-methoxy-2,3-dihydrobenzoxepin-5-yl)-3-methylpenta-2,4-diensäure;
- (2Z,4E)-5-(3,3-Dimethyl-7-methoxy-2,3-dihydrobenzoxepin-5-yl)-3-methylpenta-2,4-diensäure;
- (2E,4E)-5-(3,3-Dimethyl-7,8-dimethoxy-2,3-dihydrobenzoxepin-5-yl)-3-methylpenta-2,4-diensäure;
- (2E,4E)-5-(3,3-Dimethyl-2,3-dihydrobenzoxepin-5-yl)-3-methylpenta-2,4-diensäure;
- (2E,4E)-5-[3,3-Dimethyl-2,3-dihydro-7-(para-chlorbenzoyl)benzoxepin-5-yl]-3-methylpenta-2,4-dien]säure;
- (2E,4E)-5-(3,3-Dimethyl-7-chlor-2,3-dihydrobenzoxepin-5-yl)-3-methylpenta-2,4-diensäure;
- (2E,4E)-5-(3,3-Dimethyl-7,8-dichlor-2,3-dihydrobenzoxepin-5-yl)-3-methylpenta-2,4-diensäure;
- (2E,4E)-5-(3,3-Dimethyl-7-brom-2,3-dihydrobenzoxepin-5-yl)-3-methylpenta-2,4-diensäure;
- (2E,4E)-5-(3,3-Dimethyl-7-fluor-8-chlor-2,3-dihydrobenzoxepin-5-yl)-3-methylpenta-2,4-diensäure;
- (2E,4E)-5-(3,3-Dimethyl-7-fluor-2,3-dihydrobenzoxepin-5-yl)-3-methylpenta-2,4-diensäure;
- (2E,4E)-5-(3,3-Dimethyl-7-trifluormethyl-2,3-dihydrobenzoxepin-5-yl)-3-methylpenta-2,4-diensäure;
- (2E,4E)-5-(3,3-Dimethyl-7-phenyl-2,3-dihydrobenzoxepin-5-yl)-3-methylpenta-2,4-diensäure;
- (2E,4E)-5-(3,3,7-Trimethyl-2,3-dihydrobenzoxepin-5-yl)-3-methylpenta-2,4-diensäure;
- (2E,4E)-5-(3,3-Dimethyl-2,3-dihydrobenzoxepin-5-yl)-3-methylpenta-2,4-diensäure;
- (2E,4E)-5-(9-Methoxy-3,3-dimethyl-2,3-dihydrobenzoxepin-5-yl)-3-methylpenta-2,4-diensäure;
und ihren pharmazeutisch unbedenklichen Estern.

14. Verwendung nach einem der Ansprüche 1 bis 13, wobei die zu behandelnden mit Hyperurikämie einhergehenden Erkrankungen eine oder mehrere der Folgenden umfassen: Gicht, akute entzündliche Arthritis, tophöse Ablagerung von Uratkristallen in und um Gelenke, chronische Arthritis, Ablagerung von Uratkristallen im renalen Parenchym, Urolithiasis und damit verbundene Nierenerkrankung, die auch als Gichtnephropathie bezeichnet wird.

15. Verwendung nach einem der Ansprüche 1 bis 13, wobei die zu behandelnde Hyperurikämie primäre und sekundäre Hyperurikämie umfasst, wie beispielsweise mit Medikamenten verbundener Hyperurikämie (z. B. durch Diuretika, Immunsuppressiva oder cytotoxische Mittel) oder Hyperurikämie, die mit verschiedenen medizinischen Zuständen verbunden ist (z.B. Nephropathien, myeloproliferativen Störungen, Zuänden, die mit Insulinresistenz oder Transplantationen einhergehen).

16. Verwendung nach einem der Ansprüche 1 bis 13, zur Herstellung von Arzneimitteln für Subjekte, die Serumspiegel von Harnsäure, vor der Behandlung, von 7 mg/dL (420 µmol/1) aufweisen.

17. Verwendung nach Anspruch 16, wobei die zu behandelnden Zustände Gicht sind oder jeglicher Zustand, der durch hohe Spiegel an Harnsäure in den Gelenken oder Nieren oder einem Serumspiegel von mehr als 9 mg/dL (530 µmol/l) hervorgerufen wird.

18. Verwendung nach einem der Ansprüche 1 bis 17 zur Herstellung eines Arzneimittels, das für die Verabreichung des 2,4-Pentadiensäurederivats der Formel (I) über den oralen Weg geeignet ist.

19. Verwendung nach einem der Ansprüche 1 bis 18 zur Herstellung eines Arzneimittels zur Verabreichung einer wirksamen Menge von 2,4-Pentadiensäure oder einem Derivat gemäß Formel (I) ein- oder zweimal pro Tag.

20. Verwendung nach einem der Ansprüche 1 bis 19, wobei die Menge an diesem Pentadiensäurederivat im Wesentlichen geringer ist als die Menge, die für das relevante Derivat benötigt wird, bei Verwendung bei der Behandlung von Dyslipidämie, Atherosklerose und Diabetes.

21. Verwendung nach Anspruch 20, wobei diese Menge um mindestens 50 % geringer ist.

22. Verwendung nach Anspruch 21, wobei diese Menge um mindestens 90 % geringer ist.

23. Verwendung nach einem der Ansprüche 1 bis 20, wobei die Menge an diesem Pentadiensäurederivat 0,15 bis 4 mg/kg menschliches Körpergewicht beträgt.

24. Verwendung nach Anspruch 23, wobei diese Menge zwischen 0,3 bis 1,0 mg/kg menschliches Körpergewicht beträgt.

25. Verwendung nach einem der Ansprüche 1 bis 24, wobei dieses Derivat (2E,4E)-5-(3,3-Dimethyl-7-methoxy-2,3-dihydrobenzoxepin-5-yl)-3-methylpenta-2,4-diensäure ist oder ihre pharmazeutisch unbedenklichen Salze oder Ester, zu denen ihr Ethylester gehört.

26. Neue medizinische Zusammensetzungen, die in einer für einen Menschen unbedenklichen Träger eine wirksame Menge von mindestens einem 2,4-Pentandiensäurederivat wie in einem der Ansprüche 1 bis 13 definiert enthalten,
wobei die wirksame Menge in einer Dosis für eine eintägige Verabreichung für einen Erwachsenen 0,15 bis 4 mg/kg eines menschlichen Körpers beträgt.

27. Medizinische Zubereitungen nach Anspruch 26, wobei diese wirksame Menge zwischen 0,3 bis 1,0 mg/kg eines menschlichen Körpers beträgt.

28. Medizinische Zubereitungen nach Anspruch 26 oder 27, für orale Verabreichung formuliert.

29. Arzneimittel nach einem der Ansprüche 26 bis 28, wobei dieses Derivat (2E,4E)-5-(3,3-Dimethyl-7-methoxy-2,3-dihydrobenzoxepin-5-yl)-3-methylpenta-2,4-diensäure ist oder ihre pharmazeutisch unbedenklichen Salze oder Ester, zu denen ihr Ethylester gehört.

## Revendications

1. Utilisation d'un dérivé d'acide pentadiénoïque de formule (I) pour la préparation d'un médicament destiné à la prévention ou au traitement de l'hyperuricémie et/ou d'un(e) ou plusieurs troubles ou maladies associé(e)s, et/ou destiné à réduire le taux d'acide urique sérique d'un sujet, 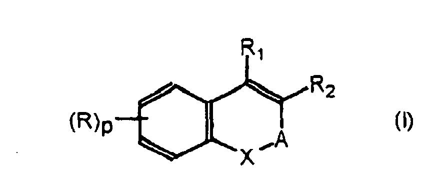 dans laquelle :
X représente O ou S ;
A représente soit le radical divalent -(CH₂)ₛ-CO-(CH₂)ₜ-, soit le radical divalent - (CH₂)ₛ-CR₃R₄-(CH₂)ₜ-,
dans lesquels radicaux s = t = 0 ou bien l'un parmi s et t a pour valeur 0 et l'autre a pour valeur 1 ;
R₄ représente un atome d'hydrogène ou un groupement alkyl (C₁-C₁₅) ;
R₁ et R₂ représentent indépendamment la chaîne Z définie ci-après ; un atome d'hydrogène ; un groupement alkyl-(C₁-C₁₈) ; un groupement alcényl(C₂-C₁₈) ; un groupement alcynyl(C₂-C₁₈) ; un groupement aryl (C₆-C₁₀) éventuellement substitué par un atome d'halogène, par un groupement alkyl-(C₁-C₅) éventuellement halogéné ou par un groupement alcoxy-(C₁-C₅) éventuellement halogéné ; ou un groupement hétéroaryl(C₄-C₁₂) mono- ou bicyclique comprenant un ou plusieurs hétéroatomes choisis parmi O, N et S qui est éventuellement substitué par un atome d'halogène, par un groupement alkyl-(C₁-C₅) éventuellement halogéné ou par un groupement alcoxy(C₁-C₅) éventuellement halogéné ;
R₃ et R₄ prennent indépendamment l'une quelconque des significations données ci-dessus pour R₁ et R₂, à l'exception de la chaîne Z ; ou bien
R₃ et R₄ forment ensemble une chaîne alkylène (C₂-C₆) éventuellement substituée par un atome d'halogène ou par alcoxy(C₁-C₅) éventuellement halogéné ;
R est choisi parmi un atome d'halogène ; un groupement cyano ; un groupement nitro ; un groupement carboxy ; un groupement alcoxy(C₁-C₁₈)carbonyle éventuellement halogéné ; un groupement Rₐ-CO-NH- ou RₐR_{b}N-CO- [dans lequel Rₐ et R_{b} représentent indépendamment alkyl(C₁-C₁₈) éventuellement halogéné ; un atome d'hydrogène ; aryl (C₆-C₁₀) ou aryl-(C₆-C₁₀)alkyl(C₁-C₅) (où les parties aryle sont éventuellement substituées par un atome d'halogène, par un groupement alkyl(C₁-C₅) éventuellement halogéné ou par un groupement alcoxy(C₁-C₅) éventuellement halogéné) ; cycloalkyl (C₃-C₁₂) éventuellement substitué par un atome d'halogène, par un groupement alkyl(C₁-C₅) éventuellement halogéné ou par un groupement alcoxy(C₁-C₅) éventuellement halogéné] ; un groupement alkyl(C₁-C₁₈) éventuellement halogéné ; alcoxy-(C₁-C₁₈) éventuellement halogéné ; et aryl(C₆-C₁₀), aryl-(C₆-C₁₀) alkyl (C₁-C₅), aryl (C₆-C₁₀) oxy, cycloalkyl (C₃-C₁₂), cycloalcényl (C₃-C₁₂), cycloalkyl (C₃-C₁₂) oxy ou cycloalcényl-(C₃-C₁₂) oxy dans lesquels les parties aryle, cycloalkyle et cycloalcényle sont éventuellement substituées par un atome d'halogène, par alkyl(C₁-C₅) éventuellement halogéné ou par alcoxy(C₁-C₅) éventuellement halogéné ;
-OH ;
p représente 0, 1, 2, 3 ou 4 ;
Z représente le radical : 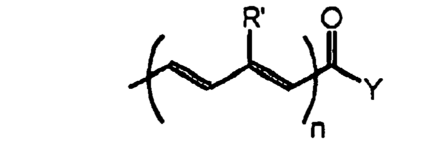 où n vaut 1 ou 2 ;
les groupements R' représentent indépendamment un atome d'hydrogène ; un groupement alkyl(C₁-C₅) ; un groupement aryl(C₆-C₁₀) éventuellement substitué par un atome d'halogène, par un groupement alkyl(C₁-C₅) éventuellement halogéné ou par alcoxy(C₁-C₅) éventuellement halogéné ; ou un groupement hétéroaryl (C₄-C₁₂) mono- ou bicyclique comprenant un ou plusieurs hétéroatomes choisis parmi O, N et S qui est éventuellement substitué par un atome d'halogène, par un groupement alkyl(C₁-C₅) éventuellement halogéné ou par un groupement alcoxy(C₁-C₅) éventuellement halogéné ;
Y représente -OH ; alcoxy(C₁-C₅) ; ou le groupement -NR_{c}R_{d} (dans lequel R_{c} et R_{d} représentent indépendamment un atome d' hydrogène ; alkyl (C₁-C₅) ; cycloalkyl (C₃-C₈) éventuellement substitué par un atome d'halogène, par alkyl-(C₁-C₅) éventuellement halogéné ou par alcoxy(C₁-C₅) éventuellement halogéné ; aryl(C₆-C₁₀) éventuellement substitué par un atome d'halogène, par alkyl(C₁-C₅) éventuellement halogéné ou par alcoxy(C₁-C₅) éventuellement halogéné ;
ou Y représente l'acide glucuronique 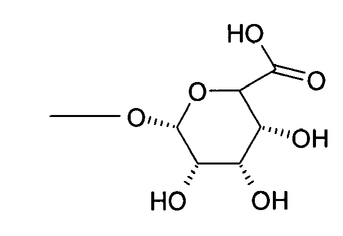 étant entendu que l'un et un seul parmi R₁ et R₂ représente la chaîne Z ;
et leurs sels pharmaceutiquement acceptables avec des acides ou des bases, ou leurs esters.

2. Utilisation selon la revendication 1, **caractérisée en ce que** A représente le radical divalent - -(CH₂)ₛ-CR₃R₄-(CH₂)ₜ- dans lequel s, t, R₃ et R₄ sont tels que définis selon la revendication 1.

3. Utilisation selon la revendication 1, **caractérisée en ce que** :
X représente O ;
A représente -CR₃R₄- ou -CH₂-CR₃R₄- dans lesquels le groupement méthylène non substitué est lié à X ;
R₁ et R₂ représentent indépendamment Z ; H ; alkyl-(C₁-C₁₅); alcényl(C₂-C₁₅); ou phényle éventuellement substitué par alkyl(C₁-C₅), alcoxy(C₁-C₅), un atome d'halogène ou -CF₃ ;
R₃ et R₄ prennent indépendamment l'une quelconque des significations données ci-dessus pour R₁ et R₂, à l'exception de Z ;
R est choisi parmi alkyl(C₁-C₉) ; alcoxy(C₁-C₅) ; phényle ou phénylcarbonyle éventuellement substitué par un atome d'halogène, alkyl (C₁-C₅), alcoxy(C₁-C₅), -CF₃ ou -OCF₃ ; un atome d'halogène ; -CF₃ et -OCF₃ ;
Z représente le radical : 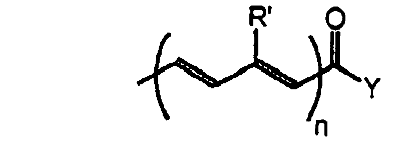 où n représente 1 ; et
R' représente alkyl (C₁-C₅) ou aryl (C₆-C₁₀) .

4. Utilisation selon l'une quelconque des revendications 1 à 3, dans laquelle :
X représente O ;
A représente -CH₂-CR₃R₄- dans lequel le groupement méthylène non substitué est lié à X ;
R₁ et R₂ représentent indépendamment Z, un atome d'hydrogène ou alkyl(C₁-C₅) ;
R₃ et R₄ prennent indépendamment l'une quelconque des significations données ci-dessus pour R₁ et R₂, à l'exception de Z ;
Z représente 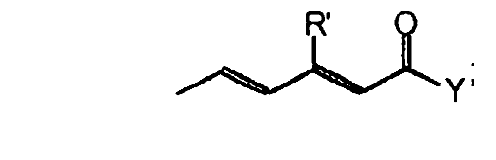 et
R' représente méthyle ou phényle.

5. Utilisation selon l'une quelconque des revendications 1 à 4, dans laquelle R₁ représente Z.

6. Utilisation selon l'une quelconque des revendications 1 à 5, dans laquelle R₂ représente un atome d'hydrogène.

7. Utilisation selon l'une quelconque des revendications 1 à 6, dans laquelle Y est alcoxy(C₁-C₅).

8. Utilisation selon l'une quelconque des revendications 1 à 6, dans laquelle :
Y représente -OH ; alcoxy(C₁-C₅) ; ou NR_{c}R_{d} dans lequel R_{c} et R_{d} sont tels que définis selon la revendication 1.

9. Utilisation selon l'une quelconque des revendications 1 à 8, dans laquelle R' est méthyle.

10. Utilisation selon l'une quelconque des revendications 1 à 9, dans laquelle R est alcoxy(C₁-C₅).

11. Utilisation selon l'une quelconque des revendications 1 à 6, dans laquelle p représente 0, 1 ou 2.

12. Utilisation selon la revendication 1, dans laquelle :
X représente O ;
A représente -CH₂-CR₃R₄- dans lequel le groupement méthylène non substitué est lié à X ;
R₁ est Z et R₂ est H ;
R₃ et R₄ représentent indépendamment un groupement alkyl (C₁-C₅) ;
R est alcoxy(C₁-C₅) ;
Z représente 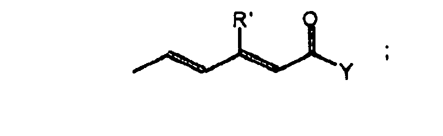
dans lequel R' représente méthyle ou phényle ; et Y représente alcoxy(C₁-C₅).

13. Utilisation selon la revendication 1, dans laquelle ledit dérivé est choisi parmi le groupe constitué par :
l'acide (2E,4E)-5-(2-pentyl-2H-1-benzopyran-3-yl)-3-méthylpenta-2,4-diénoïque ;
l'acide (2Z,4E)-5-(2-pentyl-2H-1-benzopyran-3-yl)-3-méthylpenta-2,4-diénoïque ;
l'acide (2E,4E)-5-(2,2-diméthyl-6-méthoxy-2H-1-benzopyran-3-yl)-3-méthylpenta-2,4-diénoïque ;
l'acide (2E,4E)-5-(2H-1-benzopyran-3-yl)-3-méthylpenta-2,4-diénoïque ;
l'acide (2E,4E)-5-(2,2-diméthyl-2H-1-benzopyran-3-yl)-3-méthylpenta-2,4-diénoïque ;
l'acide (2Z,4E)-5-(2,2-diméthyl-2H-1-benzopyran-3-yl)-3-méthylpenta-2,4-diénoïque ;
l'acide (2E,4E)-5-[2-(non-6-ényl)-2H-1-benzopyran-3-yl]-3-méthylpenta-2,4-diénoïque ;
l'acide (2E,4E)-5-(4-phényl-2H-1-benzopyran-3-yl)-3-méthylpenta-2,4-diénoïque ;
l'acide (2E,4E)-5-(6-nonyl-2H-1-benzopyran-3-yl)-3-méthylpenta-2,4-diénoïque ;
l'acide (2E,4E)-5-(6-phényl-2H-1-benzopyran-3-yl)-3-méthylpenta-2,4-diénoïque ;
l'acide (2E,4E)-5-(2-nonyl-2H-1-benzopyran-3-yl)-3-méthylpenta-2,4-diénoïque ;
l'acide (2E,4E)-5-(4-méthyl-2H-1-benzopyran-3-yl)-3-méthylpenta-2,4-diénoïque ;
l'acide (2Z,4E)-5-(2H-1-benzopyran-3-yl)-3-méthylpenta-2,4-diénoïque ;
l'acide (2E,4E)-5-(2-undécanyl-2H-1-benzopyran-3-yl)-3-méthylpenta-2,4-diénoïque ;
l'acide (2E,4E)-5-(2-phényl-2H-1-benzopyran-3-yl)-3-méthylpenta-2,4-diénoïque ;
l'acide (2E,4E)-5-(5-méthyl-2,3-dihydrobenzoxépin-4-yl)-3-méthylpenta-2,4-diénoïque ;
l'acide (2E,4E)-5-(3,3-diméthyl-7-méthoxy-2,3-dihydrobenzoxépin-5-yl)-3-méthylpenta-2,4-diénoïque ;
l'acide (2E,4E)-5-(2,3-dihydrobenzoxépin-4-yl)-3-méthylpenta-2,4-diénoïque ;
l'acide (2E,4E)-5-(3,3-diméthyl-7-méthoxy-2,3-dihydrobenzoxépin-5-yl)-3-phénylpenta-2,4-diénoïque ;
l'acide (2Z,4E)-5-(3,3-diméthyl-7-méthoxy-2,3-dihydrobenzoxépin-5-yl)-3-phénylpenta-2,4-diénoïque ;
l'acide (2Z,4E)-5-(3,3-diméthyl-7-méthoxy-2,3-dihydrobenzoxépin-5-yl)-3-méthylpenta-2,4-diénoïque ;
l'acide (2E,4E)-5-(3,3-diméthyl-7,8-diméthoxy-2,3-dihydrobenzoxépin-5-yl)-3-méthylpenta-2,4-diénoïque ;
l'acide (2E,4E)-5-(3,3-diméthyl-2,3-dihydrobenzoxépin-5-yl)-3-méthylpenta-2,4-diénoïque ;
l'acide (2E,4E)-5-(3,3-diméthyl-2,3-dihydro-7-(para-chlorobenzoyl)benzoxépin-5-yl)-3-méthylpenta-2,4-diénoïque ;
l'acide (2E,4E)-5-(3,3-diméthyl-7-chloro-2,3-dihydrobenzoxépin-5-yl)-3-méthylpenta-2,4-diénoïque ;
l'acide (2E,4E)-5-(3,3-diméthyl-7,8-dichloro-2,3-dihydrobenzoxépin-5-yl)-3-méthylpenta-2,4-diénoïque ;
l'acide (2E,4E)-5-(3,3-diméthyl-7-bromo-2,3-dihydrobenzoxépin-5-yl)-3-méthylpenta-2,4-diénoïque ;
l'acide (2E,4E)-5-(3,3-diméthyl-7-fluoro-8-chloro-2,3-dihydrobenzoxépin-5-yl)-3-méthylpenta-2,4-diénoïque ;
l'acide (2E,4E)-5-(3,3-diméthyl-7-fluoro-2,3-dihydrobenzoxépin-5-yl)-3-méthylpenta-2,4-diénoïque ;
l'acide (2E,4E)-5-(3,3-diméthyl-7-trifluorométhyl-2,3-dihydrobenzoxépin-5-yl)-3-méthylpenta-2,4-diénoïque ;
l'acide (2E,4E)-5-(3,3-diméthyl-7-phényl-2,3-dihydrobenzoxépin-5-yl)-3-méthylpenta-2,4-diénoïque ;
l'acide (2E,4E)-5-(3,3,7-triméthyl-2,3-dihydrobenzoxépin-5-yl)-3-méthylpenta-2,4-diénoïque ;
l'acide (2E,4E)-5-(3,3-diméthyl-2,3-dihydrobenzoxépin-5-yl)-3-méthylpenta-2,4-diénoïque ;
l'acide (2E,4E)-5-(9-méthoxy-3,3-diméthyl-2,3-dihydrobenzoxépin-5-yl)-3-méthylpenta-2,4-diénoïque ;
et leurs esters pharmaceutiquement acceptables.

14. Utilisation selon l'une quelconque des revendications 1 à 13, dans laquelle les maladies associées à l'hyperuricémie à traiter comprennent une ou plusieurs maladies parmi les suivantes : la goutte, l'arthrite inflammatoire aiguë, le dépôt tophacé de cristaux d'urate dans les articulations et autour de celles-ci, l'arthrite chronique, le dépôt de cristaux d'urate dans le parenchyme rénal, la lithiase urinaire, et la maladie rénale apparentée désignée également par néphropathie goutteuse.

15. Utilisation selon l'une quelconque des revendications 1 à 13, dans laquelle l'hyperuricémie à traiter comprend les hyperuricémies primaires et secondaires, telles que les hyperuricémies médicamenteuses (par exemple par des diurétiques, des agents immunosuppresseurs ou cytotoxiques), ou les hyperuricémies apparentées à diverses conditions médicales (par exemple les néphropathies, les troubles myélo-prolifératifs, les conditions liées à l'insulinorésistance et aux greffes) .

16. Utilisation selon l'une quelconque des revendications 1 à 13, pour la préparation de médicaments destinés aux sujets ayant des taux d'acide urique sérique, avant traitement, égaux ou supérieurs à 7 mg/dL (420 µmol/L).

17. Utilisation selon la revendication 16, dans laquelle les conditions à traiter sont la goutte ou toute condition induite par des taux élevés d'acide urique dans les articulations ou les reins, ou un taux sérique supérieur à 9 mg/dL (530 µmol/L).

18. Utilisation selon l'une quelconque des revendications 1 à 17, pour la préparation d'un médicament convenable pour l'administration du dérivé d'acide 2,4-pentadiénoïque de formule (I) par voie orale.

19. Utilisation selon l'une quelconque des revendications 1 à 18, pour la préparation d'un médicament destiné à l'administration de la quantité efficace d'acide 2,4-pentadiénoïque ou d'un dérivé selon la formule (I), une ou deux fois par jour.

20. Utilisation selon l'une quelconque des revendications 1 à 19, dans laquelle la quantité dudit dérivé d'acide pentadiénoïque est sensiblement inférieure à la quantité nécessaire pour le dérivé pertinent tel qu'utilisé dans le traitement de la dyslipidémie, de l'athérosclérose et du diabète.

21. Utilisation selon la revendication 20, dans laquelle ladite quantité est inférieure d'au moins 50%.

22. Utilisation selon la revendication 21, dans laquelle ladite quantité est inférieure d'au moins 90%.

23. Utilisation selon l'une quelconque des revendications 1 à 20, dans laquelle la quantité dudit dérivé d'acide pentadiénoïque va de 0,15 à 4 mg/kg de poids corporel humain.

24. Utilisation selon la revendication 23, dans laquelle ladite quantité va de 0,3 à 1,0 mg/kg de poids corporel humain.

25. Utilisation selon l'une des revendications 1 à 24, dans laquelle ledit dérivé est l'acide (2E,4E)-5-(3,3-diméthyl-7-méthoxy-2,3-dihydrobenzoxépin-5-yl)-3-méthylpenta-2,4-diénoïque, ou ses sels pharmaceutiquement acceptables ou leurs esters, parmi lesquels son ester éthylique.

26. Nouvelles compositions médicales comprenant, dans un véhicule acceptable pour l'homme, une quantité efficace d'au moins un dérivé d'acide 2,4-pentadiénoïque tel que défini selon l'une quelconque des revendications 1 à 13,
dans lesquelles la quantité efficace dans une dose pour une administration journalière à un adulte va de 0,15 à 4 mg/kg de corps humain.

27. Compositions médicales selon la revendication 26, dans lesquelles ladite quantité efficace va de 0,3 à 1,0 mg/kg de corps humain.

28. Compositions médicales selon la revendication 26 ou 27, formulées pour une administration orale.

29. Médicament selon l'une quelconque des revendications 26 à 28, dans lequel ledit dérivé est l'acide (2E,4E)-5-(3,3-diméthyl-7-méthoxy-2,3-dihydrobenzoxépin-5-yl)-3-méthylpenta-2,4-diénoïque, ou ses sels pharmaceutiquement acceptables ou leurs esters, parmi lesquels son ester éthylique.
